# EUROPEAN PATENT SPECIFICATION

(11) **EP 3 775 906 B1**
(45) Date of publication and mention of the grant of the patent: **30.08.2023**
(21) Application number: 19712231.0
(22) Date of filing: 27.03.2019
(51) Int. Cl.: G01N 33/574, G01N 33/68

(54) **METABOLITE-BASED BREAST CANCER DETECTION AND DIAGNOSIS**
METABOLITEN-BASIERTE ERKENNUNG UND DIAGNOSE VON BRUSTKREBS
DÉTECTION ET DIAGNOSTIC DU CANCER DU SEIN À BASE DE MÉTABOLITE

(30) Priority: 29.03.2018 EP 18164999
(43) Date of publication of application: 17.02.2021
(73) Proprietor: Burwinkel, Barbara, 69115 Heidelberg (DE)
(72) Inventor: BURWINKEL, Barbara, 69115 Heidelberg (DE); YUAN, Baowen, Beijing 100021 (CN)
(74) Representative: Grahn, Sibylla Maria
(86) International application number: PCT/EP2019/057688
(87) International publication number: WO 2019/185692

(56) References cited:
- WO-A1-2016/038157
- DATABASE EMBASE [Online] ELSEVIER SCIENCE PUBLISHERS, AMSTERDAM, NL; 1 January 2018 (2018-01-01), PAULA R ET AL: "Plasmatic levels of arginine, its precursors and acylcarnitines affected by melatonin at specific time of the day", XP002790957, Database accession no. EMB-623767100 & PAULA R ET AL: "Plasmatic levels of arginine, its precursors and acylcarnitines affected by melatonin at specific time of the day", CLINICAL CANCER RESEARCH 20180101 AMERICAN ASSOCIATION FOR CANCER RESEARCH INC. NLD, vol. 24, no. 1, Supplement 1, 1 January 2018 (2018-01-01), ISSN: 1557-3265
- JIE SHEN ET AL: "Plasma Metabolomic Profiles in Breast Cancer Patients and Healthy Controls: By Race and Tumor Receptor Subtypes", TRANSLATIONAL ONCOLOGY, vol. 6, no. 6, 1 December 2013 (2013-12-01), pages 757-765, XP055584706, United States ISSN: 1936-5233, DOI: 10.1593/tlo.13619
- MÓNICA P. CALA ET AL: "Multiplatform plasma metabolic and lipid fingerprinting of breast cancer: A pilot control-case study in Colombian Hispanic women", PLOS ONE, vol. 13, no. 2, 13 February 2018 (2018-02-13), page e0190958, XP055584709, DOI: 10.1371/journal.pone.0190958
- YONG FAN ET AL: "Human plasma metabolomics for identifying differential metabolites and predicting molecular subtypes of breast cancer", ONCOTARGET, vol. 7, no. 9, 1 March 2016 (2016-03-01), XP055584712, United States ISSN: 1949-2553, DOI: 10.18632/oncotarget.7155

## Description

The present invention relates to the use of a combination of metabolites contained in a sample of a mammalian subject in the *in vitro* or *ex vivo* diagnosis of primary breast cancer. Said combination of metabolites comprises at least the amino acids ornithine, threonine, and tryptophan, and at least one acylcarnitine, which is acetylcarnitine (C2 or AC.2.0) and optionally further component(s).

The present invention further relates to an *in vitro* or *ex vivo* method for the diagnosis of primary breast cancer, comprising providing a blood sample of a mammalian subject; determining the amount of a combination of metabolites; and comparing said amount with a control sample.

### BACKGROUND OF THE INVENTION

Breast cancer is the second most common cancer in the world and, by far, the most frequent cancer among women with an estimated 1.67 million new cancer cases diagnosed and 521,900 deaths in 2012 (Torre *et al.,* 2015). Statistically, around 25% of all cancer cases and 15% of all cancer deaths among females are breast cancer (Torre *et al.,* 2015). Though mortality rates have continued to decrease over the years due to the advances made in early diagnosis and treatment, still thousands of women die from this disease each year. Currently, 5-year survival rates for primary breast cancer is relatively high (ranging from 80% to 92% in different populations), however, survival rates decrease to less than 25% when the disease becomes metastatic (Siegel *et al.,* 2015). Most of the breast cancer related deaths are due to metastasis. Around 25-30% of primary breast cancers go on to develop metastasis, which are called metastatic breast cancers (Redig and McAllister, 2013). Approximately 10-15% of primary breast cancer patients develop metastasis within 3 years, however metastatic manifestations after 10 or more years from initial diagnosis is also recorded (Weigelt *et al*., 2005).

Conventional methods for breast cancer diagnosis include mammography followed by histopathological investigation of biopsies. However, mammography has a relatively high false negative rate (approximately 20% to 28%) (Carney *et al*., 2006), along with the risk from repeated exposure to harmful ionizing radiation. The sampling of tissue biopsies is highly invasive and just performed after strong evidence for the existence of breast cancer. Histopathology is time consuming and often prone to subjective interpretations. What's more, due to the limited availability and high heterogeneity nature of tissue biopsy, new methods need to be developed urgently to replace the traditional ones (Overman *et al.,* 2013). Liquid biopsy has the advantage of having the easiest samples to work with, including urine and blood (Gunther, 2015). Nevertheless, urine composition is highly affected by several confounding factors and more prone to microbial contaminatio. Plasma/serum have several advantages, like repeating sampling, continuously therapy monitoring and sensitivity, making them the popular sample types in breast cancer research (Diaz and Bardelli, 2014).

Since breast cancer has so many molecular/histopathological subtypes, and the specificity of each subtype leads to different disease course, the development of diagnostic and prognostic markers is the basis of personalized medicine and tailored treatment.

At present, the commonly known blood-based biomarkers for breast cancer are (Berghuis *et al*., 2017; Duffy *et al.,* 2017; Tiryakioglu *et al.,* 2017; Janni *et al.,* 2016):

| | |
|---|---|
| Proteins: | HER2, CA 15-3, CA 27.29, CEA and uPA/PAI-1; |
| Tumor-specific DNAs: | cell-free circulating DNA (cfDNA), tumor-derived DNA size, integrity, methylation and micro-RNAs; |
| Cells: | Circulating Tumor Cells (CTCs). |

Despite the fact that blood-based breast cancer biomarkers in different utility and targeting to different subtypes and stages are emerging continuously, only HER2, CA 15-3, CA 27.29, CEA and uPA/PAI-1 are approved by ASCO for clinical usage (Harris *et al.,* 2007), others do not have established utility. In addition, single biomarkers will nor suit all the cases and only work for a proportion of patients. For example, CTCs exist in only 20.2% of patients at the prediction of primary breast cancer survival (Janni *et al.,* 2016). What's more, for markers that help to detect distant metastases, they have little value in diagnosing micrometastases or locoregional recurrences (Gunther, 2015), which does not meet the criteria of good biomarkers.

Metabolomics can profile the dynamic metabolic status of living systems, which may provide clinically valuable information about metabolic alterations and help to get new insights of the pathology of breast cancer (Locasale *et al.,* 2011; Perez, 2011). Because large alterations in metabolite level may result from small changes of enzyme activity or gene mutation in living systems, the metabolome can be regarded as the integrated reflection of molecular level changes of a biological system (Denkert *et al.,* 2006).

Previous studies which used samples from breast cancer cells, tissues, urine and blood have been reported and have generated metabolites panels for various application aspects (Gunther, 2015). The metabolic state in cancer tissue often reflects hypoxic metabolism with lactate production, which is usually referred to as the Warburg effect (Koppenol *et al.,* 2011; Hsu and Sabatini, 2008). However, due to the limitations of tissue samples with high invasive and heterogeneous, urine samples easy to be affected by confounding factors and contaminated by microbiota, which make them not the perfect sample type in biomarker studying.

In breast cancer, as in other tumor types, metabolomic research remains in the experimental stage, with as yet little translation into clinical application. At present, applications of metabolomics in breast cancer research are mainly related to the discovery of biomarkers, prediction of stage, prediction of treatment effect, early detection of recurrence, prediction of recurrence risk in early breast cancer, establishment of the metastatic metabolomics signature, prediction of clinical outcome, *etc* (Hart *et al.,* 2016).

WO 2016/038157 A1 describes the use of metabolic biomarkers in assessing breast cancer in a mammalian subject, the metabolic biomarker set comprising at least one amino acid selected from glutamine, glutamate and serine, and one lipid, or glutamine and glutamate.

All in all, development of new plasma/serum biomarkers to improve primary breast cancer and metastatic breast cancer early detection and further for prognosis track, which would have higher diagnostic and life-long surveillance accuracy, is of considerable clinical importance.

Thus, there is a need for novel as well as additional diagnostic, predictive and prognostic markers that aid to distinguish early stage group, to distinguish high risk group, to monitor therapy and to predict outcome. Such markers would help to choose better screening and treatment strategies.

### SUMMARY OF THE INVENTION

The present invention solves this object as claimed in the claims.

According to the present invention this object is solved by the use of a combination of metabolites contained in a blood sample of a mammalian subject, the combination of metabolites comprising
at least the amino acids ornithine (Om), threonine (Thr), and tryptophan (Trp), and
at least one acylcarnitine, which is acetylcarnitine (C2 or AC.2.0), in the *in vitro* or *ex vivo* diagnosis of primary breast cancer comprising the detection of the presence of the metabolites in said sample and comparing it to a control sample.

According to the present invention this object is solved by an *in vitro* or *ex vivo* method for the diagnosis of primary breast cancer, comprising the following steps:
(a) providing a blood sample of a mammalian subject,
(b) determining the amount of a combination of metabolites, the combination of metabolites being as defined herein, and
(c) comparing said amount determined in (b) with a control sample

### DESCRIPTION OF THE PREFERRED EMBODIMENTS OF THE INVENTION

Before the present invention is described in more detail below, it is to be understood that this invention is not limited to the particular methodology, protocols and reagents described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art.

Concentrations, amounts, and other numerical data may be expressed or presented herein in a range format. It is to be understood that such a range format is used merely for convenience and brevity and thus should be interpreted flexibly to include not only the numerical values explicitly recited as the limits of the range, but also to include all the individual numerical values or sub-ranges encompassed within that range as if each numerical value and sub-range is explicitly recited. This same principle applies to ranges reciting only one numerical value.

Furthermore, such an interpretation should apply regardless of the breadth of the range or the characteristics being described. Also it is to be understood that ranges may differ depending on the institute/facility where the measurements are being performed, methodology of measurement, type of tissue, and technique of tissue collection.

### Metabolic biomarkers for breast cancer

As discussed above, the present invention provides the use of a combination of metabolites contained in a blood sample of a mammalian subject in the *in vitro* or *ex vivo* diagnosis of primary breast cancer.

According to the invention, the combination of metabolites comprises
*at least the amino acids* ornithine (Om), threonine (Thr), and tryptophane (Trp),
   and
*at least one acylcarnitine*, which is acetylcarnitine (C2 or AC.2.0).

Preferably, further *amino acids* selected from glutamate (Glu), aspartate (Asp), and histidine (His) can be comprised.

Preferably, further *acylcarnitines* can be comprised, which are selected from
propionylcarnitine (C3),
hexanoylcarnitine (AC.6.0),
dodecanoylcarnitine (AC. 12.0), and
hexadecenoylcarnitine (AC. 16. 1).

The combination of metabolites can furthermore comprise at least one *biogenic amine.*

Preferably, the *at least one biogenic amine* is methionine sulfoxide (Met-SO).

The combination of metabolites is particularly suitable for assessing breast cancer, wherein the assessment is for the early stage detection and diagnosis, for the prognosis, for differential diagnostic and/or for risk estimation of breast cancer.

The blood sample is preferably whole blood, plasma or serum.

The mammalian subject is preferably human.

According to the invention, the use comprises the detection of the presence of the metabolites in said sample and comparing it to a control sample.

Said control sample is preferably the sample of a healthy subject.

The metabolites from the disclosed set/combination in breast cancer patients are significantly different from the healthy controls, which is regarded as the assessment for the diagnosis and/or risk classification.

Preferably, the metabolites are measured based on a quantitative analytical method, preferably
(a) chromatography,
(b) spectroscopy,
(c) mass analyzers/ spectrometry,
and combinations thereof.
(a) Chromatography preferably comprises GC, CE, LC, HPLC, and UHPLC.
(b) Spectroscopy preferably comprises UV/Vis, IR, NIR and NMR.
(c) Mass analyzers/spectrometry preferably comprises ESI, Quadrupole Mass Analyzers, Ion Trap Mass Analyzers, TOF (Time of Flight) Mass Analyzer, Orbitrap mass analyzer, Magnetic Sector Mass Analyzer, Electrostatic Sector Mass Analyzer, Ion Cyclotron Resonance (ICR) and combinations thereof,
including single quadrupole (Q) and triple quadrupole (QqQ), QqTOF, TOF-TOF, Q-Orbitrap, APCI-QqQ, APCI-QqTOF, MALDI-QqQ, MALDI- QqTOF, and MALDI-TOF-TOF.

Preferably, the metabolites are measured using an antibody-based method, such as an ELISA. Antibodies against the metabolites or antibodies that target the combination of the metabolites of the present invention are utilized.

### Methods for diagnosis and/or prognosis

As discussed above, the present invention provides an *in vitro* or *ex vivo* method for the diagnosis of primary breast cancer.

Said method comprises the following steps:
(a) providing a blood sample of a mammalian subject,
(b) determining the amount of a combination of metabolites,
   and
(c) comparing said amount determined in (b) with a control sample

According to the invention, the combination of metabolites of step (b) is as defined herein above.

Said combination of metabolites comprises
*at least the amino acids* ornithine (Om), threonine (Thr), and tryptophane (Trp),
   and
*at least one acylcarnitine,* which is acetylcarnitine (C2 or AC.2.0).

Preferably, further *amino acids* selected from glutamate (Glu), aspartate (Asp), and histidine (His) can be comprised.

Preferably, further *acylcarnitines* can be comprised, which are selected from
propionylcarnitine (C3),
hexanoylcarnitine (AC.6.0),
dodecanoylcarnitine (AC. 12.0), and
hexadecenoylcarnitine (AC. 16. 1).

The combination of metabolites can furthermore comprise at least one *biogenic amine.*

Preferably, the *at least one biogenic amine* is methionine sulfoxide (Met-SO).

The blood sample is preferably whole blood, plasma or serum.

The mammalian subject is preferably human.

The combination of metabolites is particularly suitable for assessing breast cancer, wherein the assessment is for the early stage detection and diagnosis, for the prognosis, for differential diagnostic and/or for risk estimation.

As discussed above, the metabolites are preferably measured based on a quantitative analytical method,
preferably
(a) chromatography,
(b) spectroscopy,
(c) mass analyzers/ spectrometry,
and combinations thereof.
(a) Chromatography preferably comprises GC, CE, LC, HPLC, and UHPLC.
(b) Spectroscopy preferably comprises UV/Vis, IR, NIR and NMR.
(c) Mass analyzers/spectrometry preferably comprises ESI, Quadrupole Mass Analyzers, Ion Trap Mass Analyzers, TOF (Time of Flight) Mass Analyzer, Orbitrap mass analyzer, Magnetic Sector Mass Analyzer, Electrostatic Sector Mass Analyzer, Ion Cyclotron Resonance (ICR) and combinations thereof,
including single quadrupole (Q) and triple quadrupole (QqQ), QqTOF, TOF-TOF, Q-Orbitrap, APCI-QqQ, APCI-QqTOF, MALDI-QqQ, MALDI- QqTOF, and MALDI-TOF-TOF.

As discussed above, the metabolites are preferably measured using an antibody-based method, such as an ELISA.

Antibodies against the metabolites or antibodies that target the combination of the metabolites of the present invention are utilized.

### Further description of preferred embodiments

So far, metabolites which were identified to be associated with breast cancer cover lipids, amino acids, proteins and saccharides (see e.g. Tenori *et al.,* 2015; Kuhn *et al.,* 2016; Fan *et al*., 2016; Hadi *et al.,* 2017; Zhou *et al.,* 2017). However, breast cancer metabolomics research is still in its infancy, and one of the main tasks was to explore reproducible and better performance metabolic biomarkers.

The inventors have now identified 37 metabolites that significantly differed between primary breast cancer and healthy controls in discovery study, among which they identified 18 metabolites that also significantly differ in the validation study for p180 kit (see Table 3). Similarly, the inventors have further identified 96 metabolites that significantly differed between primary breast cancer and healthy controls in discovery study, among which they identified 16 metabolites that also significantly differ in the validation study for p400 kit (see Table 4).

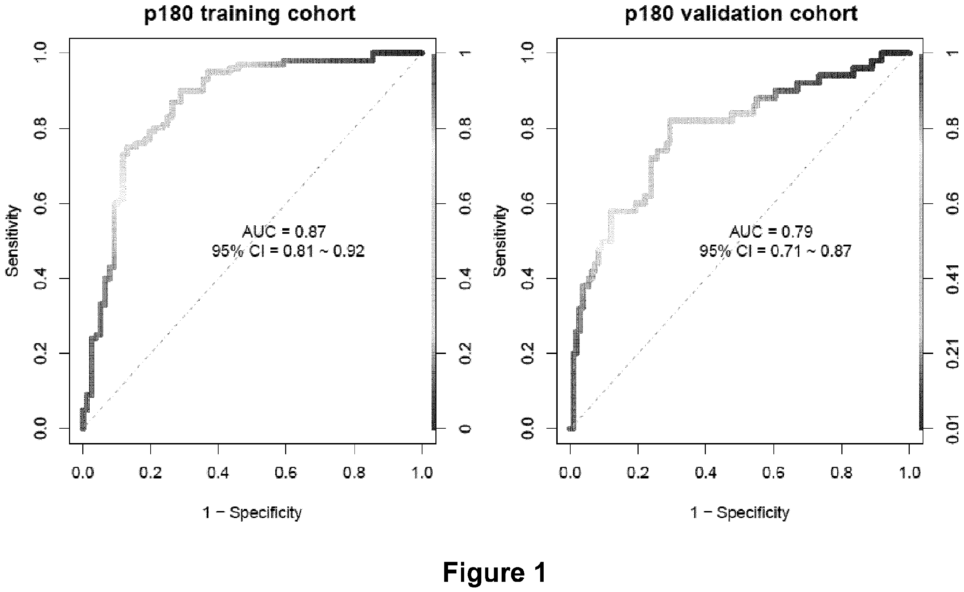

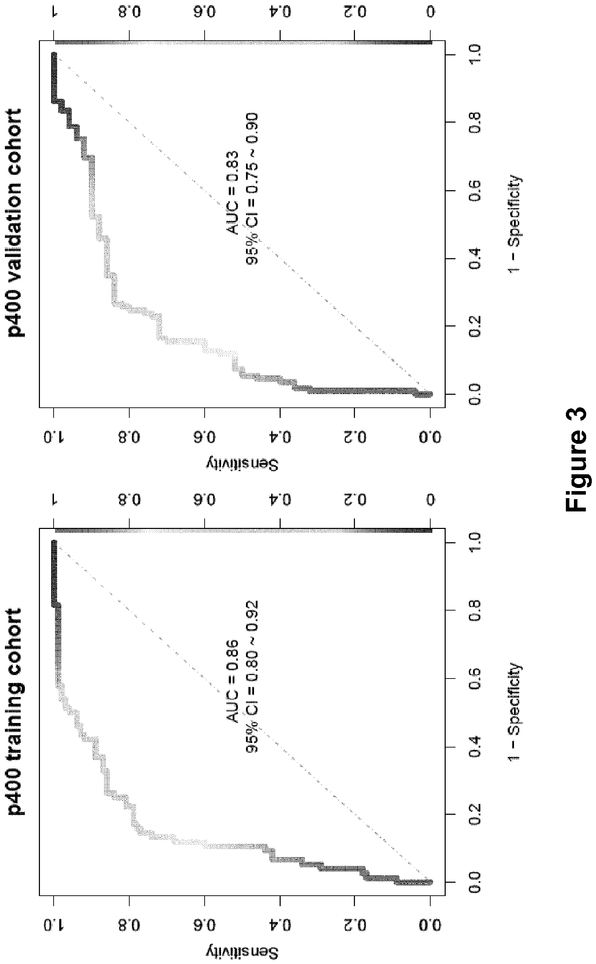

ROC analysis was built by penalized LASSO logistic regression model (with penalty parameter tuning conducted by 10-fold cross-validation), was used to compute the least redundant and most informative panel of metabolites that can discriminate primary breast cancer and healthy control groups. A good discriminatory accuracy using seven metabolites (Glu, Orn, The Trp, Met-SO, C2, C3) with AUC = 0.79 (95% CI: 0.71 - 0.87) for LASSO logistic regression model was built (Figure 1). Furthermore, a good discriminatory accuracy using nine metabolites (Asp, His, Orn, Thr, Trp, AC.2.0, AC.6.0, AC. 12.0) with AUC = 0.83 (95% CI: 0.75 - 0.90) for LASSO logistic regression model was built (Figure 3). In both, four metabolites are common, namely Orn, Thr, Trp, C2.

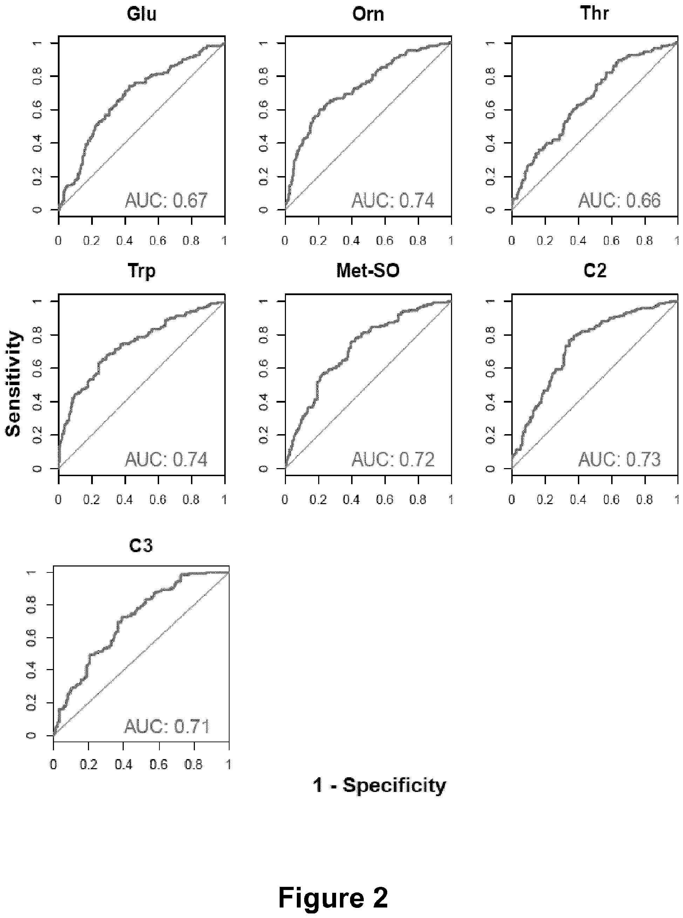

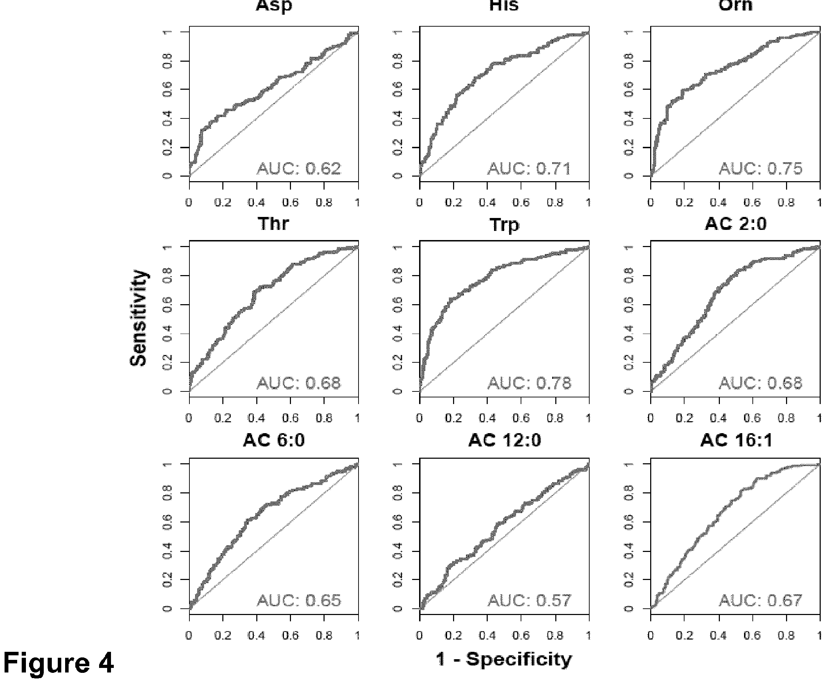

In addition, individual metabolites also have ability in distinguishing primary breast cancer from healthy control groups in both kits, though the performance is not as high as the combined panel (Figure 2 and 4).

**Table 3. Significantly different metabolites between primary breast cancer and healthy controls in both discovery and validation cohorts of p180 kit.**

| Metabolites | Discovery cohort | | | Validation cohort | | |
|---|---|---|---|---|---|---|
| | P | FDR | FC | P | FDR | FC |
| Ala | 4.59E-04 | 3.33E-03 | -1.13 | 1.59E-03 | 1.45E-02 | -1.12 |
| Asn | 2.45E-04 | 2.16E-03 | -1.16 | 7.46E-05 | 2.48E-03 | -1.21 |
| Glu | 1.03E-03 | 6.45E-03 | -1.33 | 7.74E-04 | 9.41E-03 | -1.31 |
| His | 1.60E-04 | 1.83E-03 | -1.11 | 3.14E-03 | 2.70E-02 | -1.11 |
| Leu | 3.99E-03 | 1.67E-02 | -1.14 | 5.10E-03 | 3.72E-02 | -1.13 |
| Lys | 1.52E-04 | 1.83E-03 | -1.15 | 1.48E-03 | 1.44E-02 | -1.13 |
| Met | 2.67E-03 | 1.27E-02 | -1.17 | 8.48E-05 | 2.48E-03 | -1.20 |
| Orn | 6.26E-06 | 1.73E-04 | -1.24 | 1.20E-05 | 8.79E-04 | -1.27 |
| Phe | 2.15E-03 | 1.10E-02 | -1.10 | 6.95E-03 | 4.23E-02 | -1.09 |
| Thr | 2.51E-04 | 2.16E-03 | -1.18 | 6.72E-03 | 4.23E-02 | -1.18 |
| Trp | 2.04E-06 | 1.22E-04 | -1.21 | 1.83E-05 | 8.91E-04 | -1.20 |
| Tyr | 2.59E-05 | 5.95E-04 | -1.21 | 6.04E-03 | 4.20E-02 | -1.11 |
| Val | 3.26E-04 | 2.50E-03 | -1.12 | 6.79E-03 | 4.23E-02 | -1.08 |
| Kynurenine | 2.79E-04 | 2.27E-03 | -1.22 | 1.05E-03 | 1.10E-02 | -1.17 |
| Met.SO | 2.65E-06 | 1.22E-04 | -1.46 | 1.27E-04 | 2.65E-03 | -1.31 |
| C14.1 | 2.40E-04 | 2.16E-03 | 1.19 | 2.91E-04 | 4.47E-03 | 1.35 |
| C2 | 1.56E-07 | 2.16E-05 | 1.46 | 3.06E-04 | 4.47E-03 | 1.41 |
| C3 | 4.18E-06 | 1.44E-04 | -1.31 | 7.59E-04 | 9.41E-03 | -1.19 |

**Table 4. Significantly different metabolites between primary breast cancer and healthy controls in both discovery and validation cohorts of p400 kit.**

| Metabolites | Discovery cohort | | | Validation cohort | | |
|---|---|---|---|---|---|---|
| | P | FDR | FC | P | FDR | FC |
| Asn | 2.37E-06 | 3.81E-05 | -1.18 | 2.72E-05 | 8.55E-04 | -1.20 |
| Asp | 1.32E-05 | 1.45E-04 | -1.32 | 1.53E-06 | 1.04E-04 | -1.37 |
| His | 2.47E-04 | 1.50E-03 | -1.11 | 2.91E-03 | 4.01E-02 | -1.11 |
| Met | 2.12E-04 | 1.32E-03 | -1.18 | 2.05E-04 | 5.07E-03 | -1.20 |
| Orn | 1.15E-06 | 2.34E-05 | -1.24 | 2.37E-04 | 5.36E-03 | -1.29 |
| Thr | 3.15E-05 | 2.84E-04 | -1.19 | 2.09E-03 | 3.35E-02 | -1.18 |
| Trp | 4.72E-09 | 4.50E-07 | -1.21 | 2.86E-07 | 5.41E-05 | -1.20 |
| AC.2.0. | 3.97E-08 | 2.27E-06 | 1.45 | 5.15E-06 | 2.80E-04 | 1.40 |
| AC.4.0.OH. | 9.97E-05 | 6.95E-04 | 1.44 | 2.95E-03 | 4.01E-02 | 2.54 |
| AC.6.0. | 6.48E-03 | 2.35E-02 | 1.37 | 3.50E-03 | 4.53E-02 | 1.33 |
| AC.12.0. | 8.95E-04 | 4.49E-03 | 1.28 | 1.92E-03 | 3.35E-02 | 1.29 |
| AC.12.1. | 5.98E-05 | 4.75E-04 | 1.40 | 2.83E-05 | 8.55E-04 | 1.45 |
| AC.14.0.OH. | 2.41E-03 | 1.03E-02 | 1.40 | 8.04E-04 | 1.68E-02 | 1.60 |
| AC.14.1. | 7.72E-08 | 3.14E-06 | 1.52 | 1.38E-06 | 1.04E-04 | 1.51 |
| AC.14.2. | 8.04E-05 | 5.91E-04 | 1.52 | 1.08E-03 | 2.11E-02 | 1.40 |
| AC.16.1. | 1.61E-07 | 5.11E-06 | 1.45 | 3.98E-07 | 5.41E-05 | 1.44 |

| | | | | | | |
|---|---|---|---|---|---|---|
| FDR: Logistic regression analysis with adjustment for multiple testing by controlling for false discovery rate (Benjamini-Hochberg method); | | | | | | |

Detailed information of the metabolites are shown in Tables 1 and 2 below.

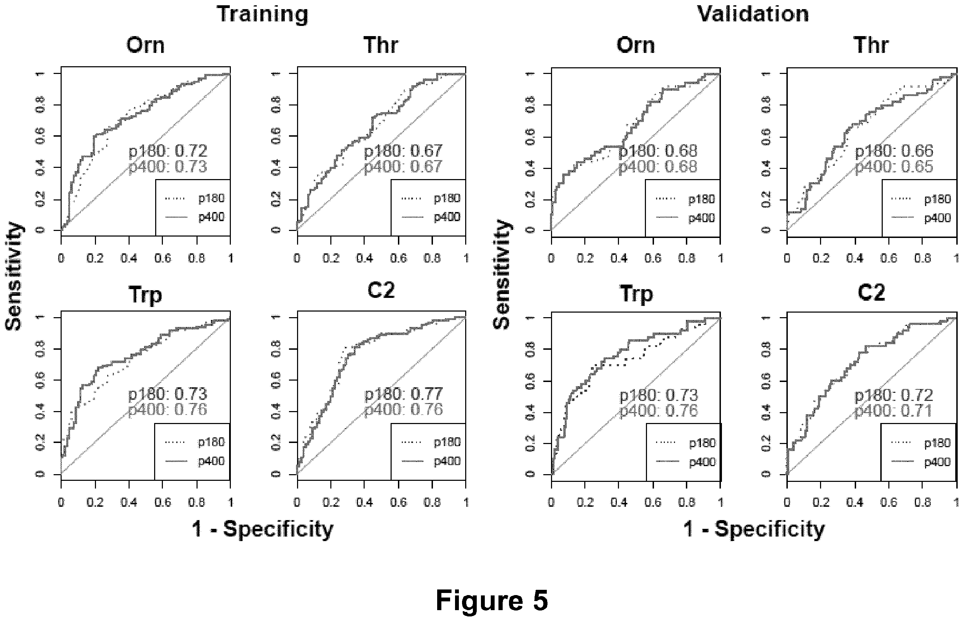

Regarding the four common metabolites within two kits, individual metabolites of these four have ability in distinguishing primary breast cancer from healthy control groups. For both training and validation dataset in p180 kit and p400 kit (Figure 5).

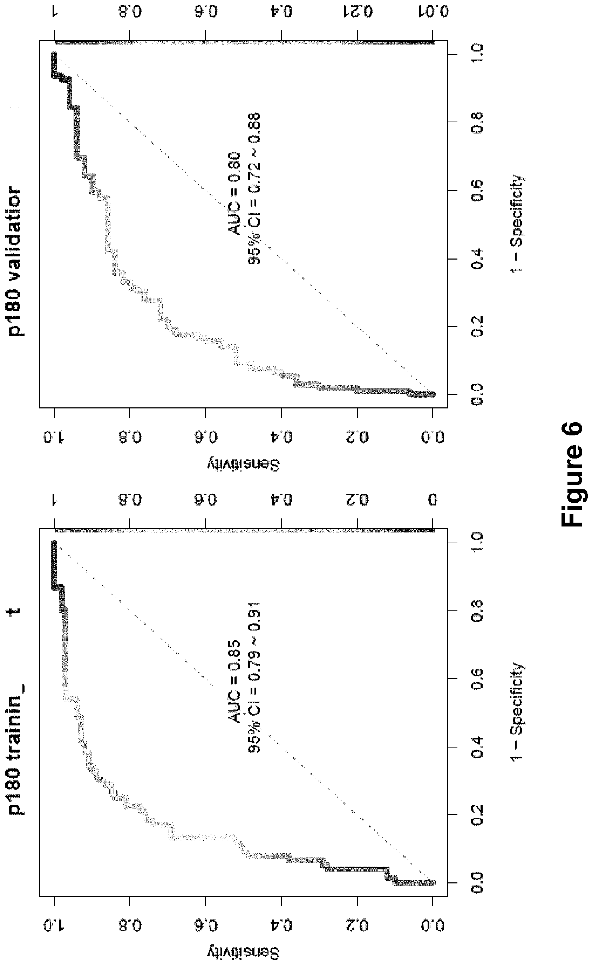

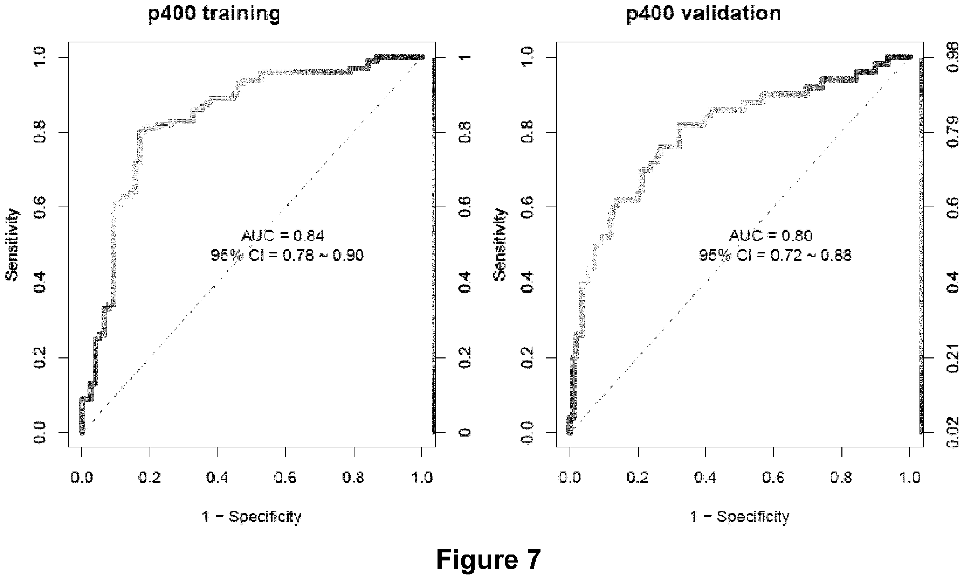

ROC curves based on logistic regression model have shown that the panel of four combined metabolites has a higher performance in discriminating primary breast cancer from healthy controls in p180 kit, with AUC=0.85 (95% CI: 0.79-0.91) in discovery cohort and AUC=0.80 (95% CI: 0.72-0.88) in validation cohort (Figure 6). Similarly, a good performance in discriminating primary breast cancer from healthy controls in p400 kit, with AUC=0.84 (95% CI: 0.78-0.90) in discovery cohort and AUC=0.80 (95% CI: 0.72-0.88) in validation cohort, were also investigated (Figure 7).

In addition, the classification between primary breast cancer hormone subtypes and healthy controls were also observed with the four common metabolites. ROC curves based on logistic regression model have shown that the panel of four combined metabolites has a good performance in discriminating primary breast cancer subtypes from healthy controls in p180 kit, with AUC=0.85 (95% CI: 0.79-0.91) in discovery cohort and AUC=0.80 (95% CI: 0.72-0.88) in validation cohort for luminal and healthy controls (Figure 8); AUC=0.82 (95% CI: 0.67-0.96) in discovery cohort and AUC=0.81 (95% CI: 0.50-1.00) in validation cohort for Her2 positive and healthy controls (Figure 9); and AUC=0.83 (95% CI: 0.70-0.96) in discovery cohort and AUC=0.82 (95% CI: 0.73-0.92) in validation cohort for triple negative breast cancer (TNBC) and healthy controls (Figure 10). Similarly, a good performance in discriminating primary breast cancer hormone subtypes from healthy controls in p400 kit was also observed, with AUC=0.86 (95% CI: 0.79-0.93) in discovery cohort and AUC=0.78 (95% CI: 0.69-0.87) in validation cohort for luminal and healthy controls (Figure 11); AUC=0.81 (95% CI: 0.65-0.97) in discovery cohort and AUC=0.81 (95% CI: 0.50-1.00) in validation cohort for Her2 positive and healthy controls (Figure 12), and AUC=0.81 (95% CI: 0.68-0.94) in discovery cohort and AUC=0.84 (95% CI: 0.75-0.93) in validation cohort for triple negative breast cancer (TNBC) and healthy controls (Figure 13).

In the case of any lipids it should be noted that, the detected signal is a sum of several isobaric lipids with the same molecular weight (±0.5 Da range) within the same class. This is because of the limitation of the mass resolution in the preferably employed MS/MS measurements. For example, the signal of PC aa C36:6 can arise from different lipid species that have different fatty acid composition (e.g. PC 16:1/20:5 versus PC 18:4/18:2), various positioning of fatty acids sn-1/sn-2 (e.g. PC 18:4/18:2 versus PC18:2/18:4) and different double bond positions and stereochemistry in those fatty acid chains (e.g. PC(18:4(6Z,9Z,12Z,15Z)/18:2(9Z,12Z)) versus PC(18:4(9Z,11Z,13Z,15Z)/18:2(9Z,12Z)).

The following examples and drawings illustrate the present invention without, however, limiting the same thereto.

### BRIEF DESCRIPTION OF THE FIGURES

**Figure 1****:** *Receiver operating characteristics (ROC) curve of the classifier for the differentiation between healthy controls and primary breast cancer patients of p180 kit.*
   Performance on the discovery cohort is shown to the left whereas the performance on the validation cohort is shown to the right. The continuous line represents the discrimination power of the classifier. Corresponding AUC and 95% confidence interval (CI) are shown. ROC curves are colorized according to the cut-off, the scale of which is represented at the right side of the plot. The x-axis and y-axis show the false positive rate (1-specificity) and the true positive rate (sensitivity), respectively. The dash-dotted diagonal line indicates no discrimination power, i.e. random classification.
   The multiparametric panel based on panalized LASSO logistic regression model for p180 kit with 7 variables has best performance: Glu, Orn, Thr, Trp, Met-SO, C2, C3.
**Figure 2****:** *Receiver operating characteristics (ROC) curve of the classifier for the differentiation between healthy controls and primary breast cancer patients of p180 kit with single feature.*
   Performance on p180 kit show the comparison between primary breast cancer and healthy controls with single features. The corresponding features are indicated in the title of plot. The continuous (red) line represents the discrimination power of the classifier. Corresponding AUC are shown. The x-axis and y-axis show the false positive rate (1-specificity) and the true positive rate (sensitivity), respectively. The diagonal line indicates no discrimination power, i.e. random classification.
**Figure 3****:** *Receiver operating characteristics (ROC) curve of the classifier for the differentiation between healthy controls and primary breast cancer patients of p400 kit.* Performance on the discovery cohort is shown to the left whereas the performance on the validation cohort is shown to the right. The multiparametric panel based on panalized LASSO logistic regression model for p400 kit with 9 variables has best performance: Asp, His, Orn, Thr, Trp, AC.2.0., AC.6.0., AC.12.0., AC.16.1. Among which, four metabolites are common in both kits: Orn, Thr, Trp and C2.
**Figure 4****:** *Receiver operating characteristics (ROC) curve of the classifier for the differentiation between healthy controls and primary breast cancer patients of p400 kit with single features.*
   The corresponding features are indicated in the title of plot.
**Figure 5****:** *Receiver operating characteristics (ROC) curve of the classifier for the differentiation between healthy controls and primary breast cancer patients of p180 kit and p400 kit with the four overlapping single features.*
   Performance on the discovery cohort is shown to the left, performance on the validation cohort is shown to the right. To show the comparison between primary breast cancer and healthy controls. The dotted line represents the discrimination power of the classifier in p180 kit, while the continuous line represents p400 kit. Corresponding AUC are shown. The corresponding features are indicated in the title of plot.
**Figure 6****:** *Receiver operating characteristics (ROC) curve of the classifier for the differentiation between healthy controls and primary breast cancer patients of p180 kit.* The multiparametric panel based on logistic regression model with the four overlapping features: Orn, Thr, Trp, C2. Performance on the discovery cohort is shown to the left, performance on the validation cohort is shown to the right.
**Figure 7****:** *Receiver operating characteristics (ROC) curve of the classifier for the differentiation between healthy controls and primary breast cancer patients of p400 kit.* The multiparametric panel based on logistic regression model with the four overlapping features: Orn, Thr, Trp, C2. Performance on the discovery cohort is shown to the left, performance on the validation cohort is shown to the right.

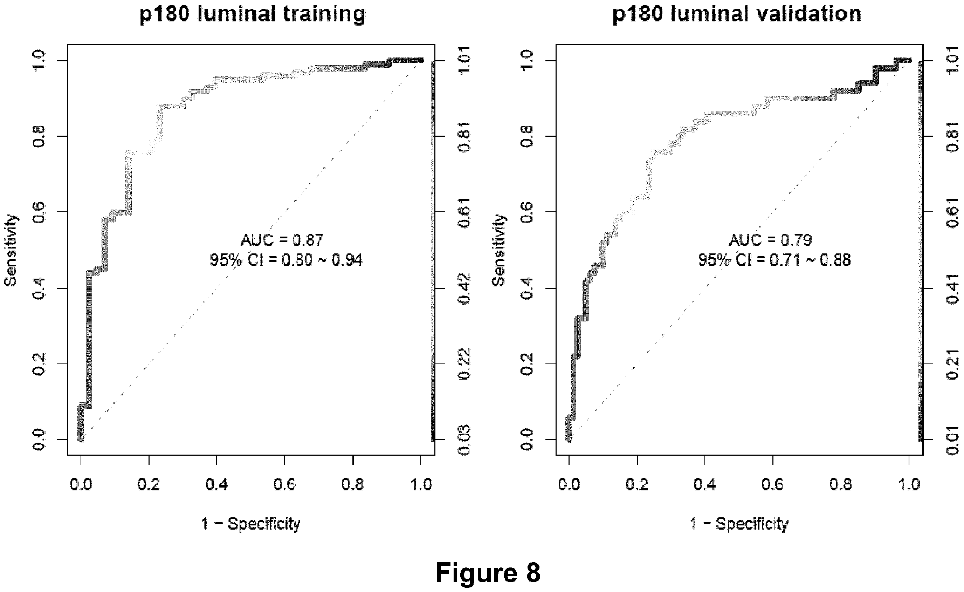

**Figure 8****:** *Receiver operating characteristics (ROC) curve of the classifier for the differentiation between healthy controls and primary breast cancer patient Luminal subtype of p180 kit.*
   The multiparametric panel based on logistic regression model with the four overlapping features: Orn, Thr, Trp, C2. Performance on the discovery cohort is shown to the left, performance on the validation cohort is shown to the right.

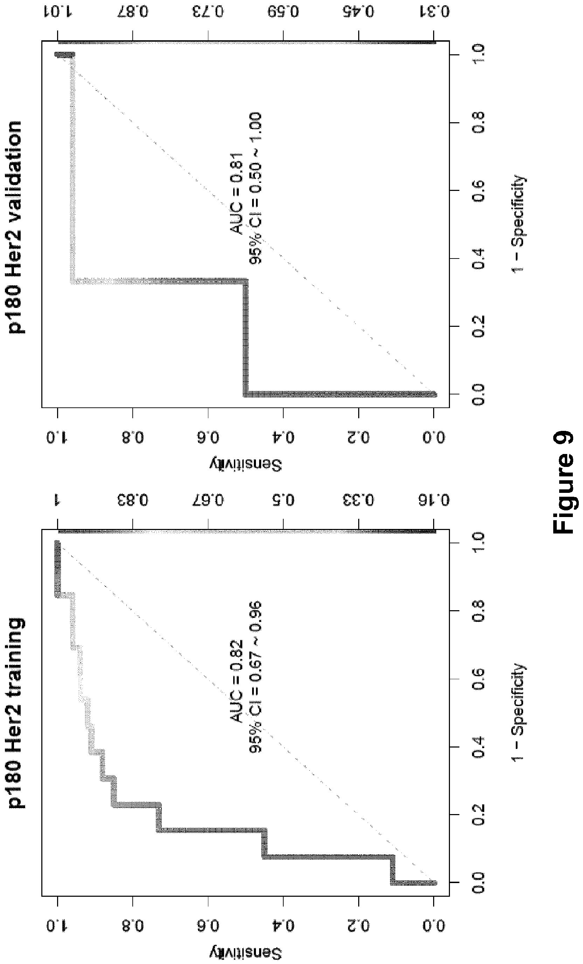

**Figure 9****:** *Receiver operating characteristics (ROC) curve of the classifier for the differentiation between healthy controls and primary breast cancer patient Her2 positive subtype of p180 kit.*
   The multiparametric panel based on logistic regression model with the four overlapping features: Orn, Thr, Trp, C2. Performance on the discovery cohort is shown to the left, performance on the validation cohort is shown to the right.

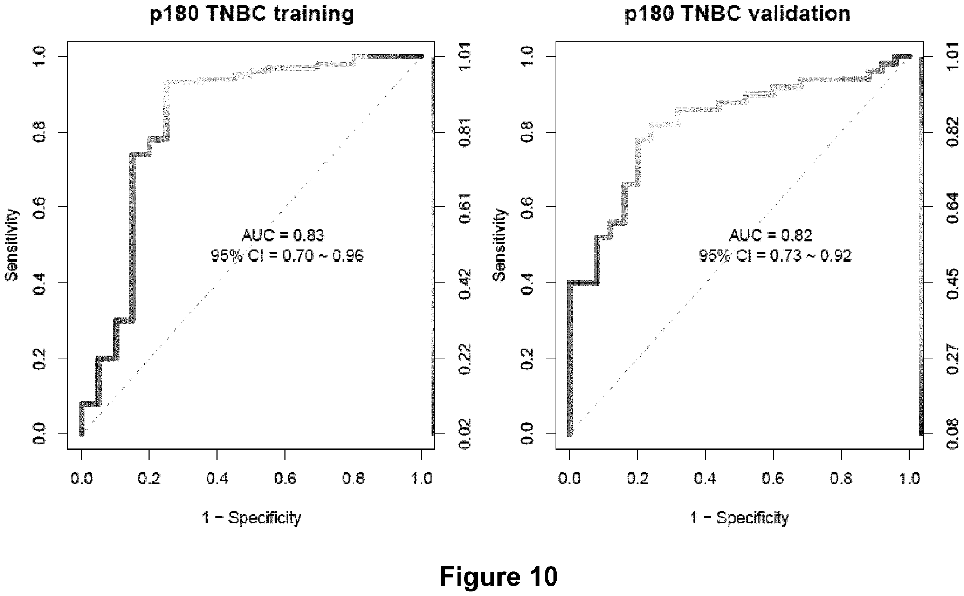

**Figure 10****:** *Receiver operating characteristics (ROC) curve of the classifier for the differentiation between healthy controls and primary breast cancer patient triple negative subtype of p180 kit.*
   The multiparametric panel based on logistic regression model with the four overlapping features: Orn, Thr, Trp, C2. Performance on the discovery cohort is shown to the left, performance on the validation cohort is shown to the right.

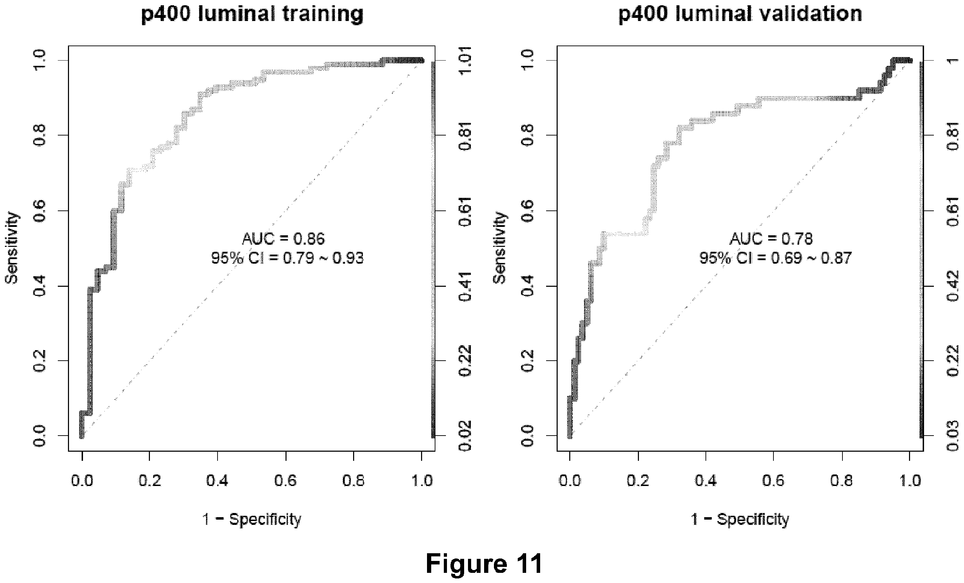

**Figure 11****:** *Receiver operating characteristics (ROC) curve of the classifier for the differentiation between healthy controls and primary breast cancer patient Luminal subtype of p400 kit.*
   The multiparametric panel based on logistic regression model with the four overlapping features: Orn, Thr, Trp, C2. Performance on the discovery cohort is shown to the left, performance on the validation cohort is shown to the right.

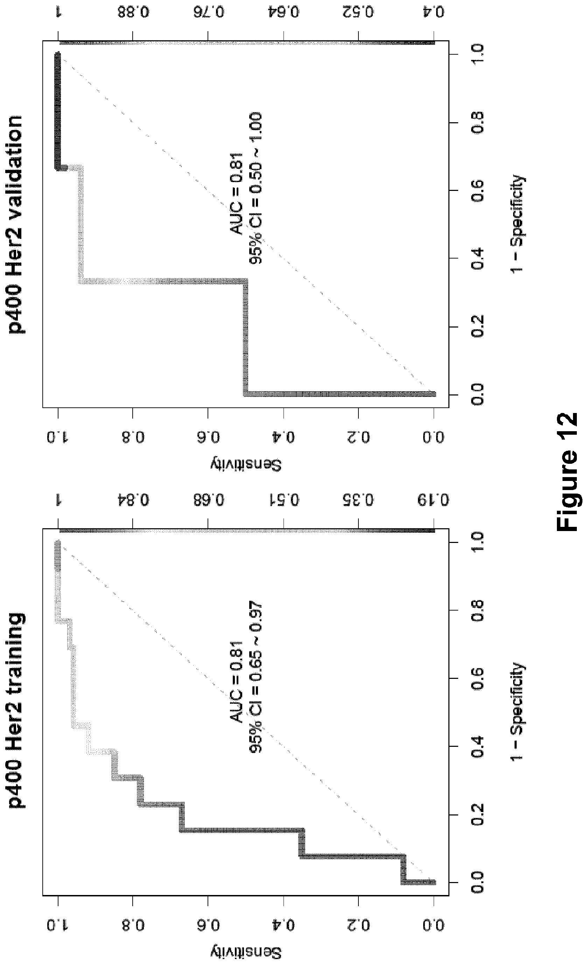

**Figure 12****:** *Receiver operating characteristics (ROC) curve of the classifier for the differentiation between healthy controls and primary breast cancer patient Her2 positive subtype of p400 kit.*
   The multiparametric panel based on logistic regression model with the four overlapping features: Orn, Thr, Trp, C2. Performance on the discovery cohort is shown to the left, performance on the validation cohort is shown to the right.

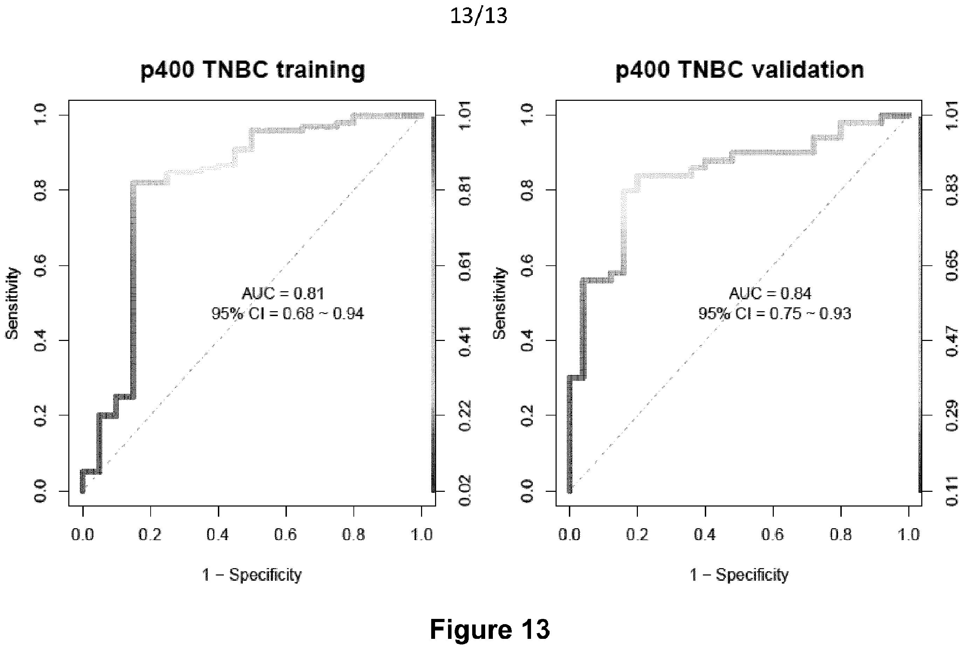

**Figure 13****:** *Receiver operating characteristics (ROC) curve of the classifier for the differentiation between healthy controls and primary breast cancer patient triple negative subtype of p400 kit.*
   The multiparametric panel based on logistic regression model with the four overlapping features: Orn, Thr, Trp, C2. Performance on the discovery cohort is shown to the left, performance on the validation cohort is shown to the right.

### EXAMPLES

### 1. Materials and Methods

### 1.1 Clinic blood sample collection and metabolites measurement

In this research project, samples from three types of study cohorts were investigated; all studies were approved by the Ethical Committee. Clinical data. detailed questionnaire data and follow up information were available for all patients. All subjects were female and of Caucasian origin thus matched for gender and ethnicity. Written consent was obtained from all participants prior to phlebotomy.

*Primary breast cancer patients*: The primary breast cancer cohort consisted of patients with sporadic and initial diagnosis of breast cancer. Blood was collected from them before they underwent any therapy or surgery. Histopathological features were determined from tumour tissue obtained from both the initial biopsy and surgical resection. In case of discrepancy between the two. the results of the latter were considered valid. This cohort included 80 patients for discovery study and 109 patients for validation study.

*Breast cancer follow up patients*: Blood samples were collected during follow-up visits in intervals of six months after initial therapy of the primary breast cancer for every patient. In total. 171 samples from 28 recurrent patients and 60 non-recurrent patients at two follow-up time points were included in this study. Samples from the first time points were mainly taken after the completion of therapy. and early before the recurrence. For the 23 non-recurrent patients (five samples missing at the second time points). samples at the second time points were taken at the time right before relapse. For the 60 non-recurrent patients. samples were all taken at the third follow-up time point.

*Healthy control individuals*: Healthy individuals with no history of malignant diseases. with no auto-immune disease and no current inflammatory condition (based on self-report). In addition to the blood sample. the volunteers were asked to fill in a questionnaire regarding their lifestyles. This cohort included 100 patients for discovery study and 50 patients for validation study.

Metabolite quantification by targeted metabolomics was done with the AbsoluteIDQ^{®} p180 Kit (herein referred to as "p180 kit") and the AbsoluteIDQ^{®} p400 HR Kit (herein referred to as "p400 kit") according to the manufacturer's instructions (Biocrates Life Sciences AG. Austria). In total, 6 classes of metabolites were measured for each sample in the p180 kit, namely:
- acylcarnitines,
- amino acids,
- biogenic amines,
- monosaccharides,
- sphingolipis, and
- glycerophospholipids.

In total, 12 classes of metabolites were measured for each sample in the p400 kit, namely:
- acylcarnitines,
- amino acids,
- biogenic amines,
- monosaccharides,
- sphingomyelins,
- diglycerides,
- triglycerides,
- lysophosphatidylcholines,
- phosphatidylcholines,
- ceramides, and
- cholesteryl esters.

For details, see Tables 1 and 2 below.

### 1.2 Quality control

Coefficient of variability of 5 triplicated samples were calculated to check the robustness of the results. Then sample quality control and metabolite quality control were performed according to the concentration of specific metabolites and limit of detection (LOD). respectively. Concentration values below LOD were replaced by imputed values afterwards

### 1.3 Biomarker candidates identification

Potential co-founders like age, stage, hormone status and other characteristics were identified and adjusted. Then multiple pairwise comparisons or regressions between sample groups were performed to select significantly variable metabolites as biomarker candidates. Multiple testing adjustments were used in all testing with Bonferroni correction to control the false discovery rate (FDR) at the level of 0.05.
1) The metabolite profiles of each group were compared to each other to identify specifically and significantly different metabolites. to aid us in distinguishing:
   - Primary breast cancer patients from healthy controls,
   - Primary breast cancer hormone subtype patients,
   - Breast cancer relapse patients from non-relapse patients at different time points.
2) Correlate metabolite profile in primary breast cancer patients with known molecular and histopathological subtypes;
3) Correlate metabolite profile with clinical data such as menopause, grading, disease free intervals, site of metastasis. etc.

### 1.4 ROC analysis: diagnostic value of candidate metabolites

Multiple machine learning methods including Support Vector Machine (SVM), elastic net model, Lasso regression, Generalized Boosted Regression Models (GBM), Boosted Logistic Regression (Logit), Classification And Regression Trees (CART) and Random Forest (RF) were employed to construct prediction model using previously identified significant metabolites. Their performance was evaluated using receiver operating characteristic curve (ROC).

### Table 1. List of metabolites measured in Biocrates p180 kit:

**Table 1a. Amino Acids and Biogenic Amines**

| **Abbreviation** | **Analyte** |
|---|---|
| Ala | Alanine |
| Arg | Arginine |
| Asn | Asparagine |
| Asp | Aspartate |
| Cit | Citrulline |
| Gln | Glutamine |
| Glu | Glutamate |
| Gly | Glycine |
| His | Histidine |
| Ile | Isoleucine |
| Leu | Leucine |
| Lys | Lysine |
| Met | Methionine |
| Orn | Ornithine |
| Phe | Phenylalanine |
| Pro | Proline |
| Ser | Serine |
| Thr | Threonine |
| Trp | Tryptophan |
| Tyr | Tyrosine |
| Val | Valine |
| Ac-Orn | Acetylornithine |
| ADMA | Asymmetric dimethylarginine |
| SDMA | Symmetric dimethylarginine |
| total DMA | Total dimethylarginine |
| alpha-AAA | alpha-Aminoadipic acid |
| Carnosine | Carnosine |
| Creatinine | Creatinine |
| Histamine | Histamine |
| Kynurenine | Kynurenine |
| Met-SO | Methionine-sulfoxide |
| Nitro-Tyr | Nitro-tyrosine |
| OH-Pro | Hydroxy-proline |
| PEA | Phenylethylamine |
| Putrescine | Putrescine |
| Sarcosine | Sarcosine |
| Serotonin | Serotonin |
| Spermidine | Spermidine |
| Spermine | Spermine |
| Taurine | Taurine |
| Dopamine | Dopamine |
| DOPA | dihydroxyphenylalanine |

**Table 1b. Acylcarnitines**

| **Abbreviation** | **Analyte** |
|---|---|
| C0 | Carnitine |
| C2 | Acetylcarnitine |
| C3 | Propionylcarnitine |
| C3:1 | Propenoylcarnitine |
| C3-OH | Hydroxypropionylcarnitine |
| C4 | Butyrylcarnitine |
| C4:1 | Butenylcarnitine |
| C4-OH (C3-DC) | Hydroxybutyrylcarnitine |
| C5 | Valerylcarnitine |
| C5:1 | Tiglylcarnitine |
| C5 :1-DC | Glutaconylcamitine |
| C5-DC (C6-OH) | Glutarylcarnitine* (Hydroxyhexanoylcarnitine) |
| C5-M-DC | Methylglutarylcamitine |
| C5-OH (C3-DC-M) | Hydroxyvalerylcarnitine (Methylmalonylcarnitine) |
| C6 (C4:1-DC) | Hexanoylcarnitine (Fumarylcarnitine) |
| C6:1 | Hexenoylcarnitine |
| C7-DC | Pimelylcarnitine |
| C8 | Octanoylcarnitine |
| C9 | Nonaylcarnitine |
| C10 | Decanoylcarnitine |
| C10:1 | Decenoylcarnitine |
| C10:2 | Decadienylcarnitine |
| C12 | Dodecanoylcarnitine |
| C12:1 | Dodecenoylcarnitine |
| C12-DC | Dodecanedioylcarnitine |
| C14 | Tetradecanoylcarnitine |
| C14:1 | Tetradecenoylcarntine |
| C14:1-OH | Hydroxytetradecenoylcarnitine |
| C14:2 | Tetradecadienylcarnitine |
| C14:2-OH | Hydroxytetradecadienylcarnitine |
| C16 | Hexadecanoylcarnitine |
| C16:1 | Hexadecenoylcarnitine |
| C16:1-OH | Hydroxyhexadecenoylcarnitine |
| C16:2 | Hexadecadienylcarnitine |
| C16:2-OH | Hydroxyhexadecadienylcarnitine |
| C16-OH | Hydroxyhexadecanoylcarnitine |
| C18 | Octadecanoylcarnitine |
| C18:1 | Octadecenoylcarnitine |
| C18:1-OH | Hydroxyoctadecenoylcarnitine |
| C18:2 | Octadecadienylcarnitine |

**Table 1c. Hexoses**

| **Abbreviation** | **Analyte** |
|---|---|
| H1 | Hexose |

**Table 1d. Sphingolipids**

| **Abbreviation** | **Analyte** |
|---|---|
| SM (OH) C14:1 | Hydroxysphingomyelin with acyl residue sum C14:1 |
| SM (OH) C16:1 | Hydroxysphingomyelin with acyl residue sum C16:1 |
| SM (OH) C22:1 | Hydroxysphingomyelin with acyl residue sum C22:1 |
| SM (OH) C22:2 | Hydroxysphingomyelin with acyl residue sum C22:2 |
| SM (OH) C24:1 | Hydroxysphingomyelin with acyl residue sum C24:1 |
| SM C16:0 | Sphingomyelin with acyl residue sum C16:0 |
| SM C16:1 | Sphingomyelin with acyl residue sum C16:1 |
| SM C18:0 | Sphingomyelin with acyl residue sum C18:0 |
| SM C18:1 | Sphingomyelin with acyl residue sum C18:1 |
| SM C20:2 | Sphingomyelin with acyl residue sum C20:2 |
| SM C22:3 | Sphingomyelin with acyl residue sum C22:3 |
| SM C24:0 | Sphingomyelin with acyl residue sum C24:0 |
| SM C24:1 | Sphingomyelin with acyl residue sum C24:1 |
| SM C26:0 | Sphingomyelin with acyl residue sum C26:0 |
| SM C26:1 | Sphingomyelin with acyl residue sum C26:1 |

**Table 1e. Glycerophospholipids**

| **Abbreviation** | **Analyte** |
|---|---|
| lysoPC a C14:0 | Lysophosphatidylcholine with acyl residue C14:0 |
| lysoPC a C16:0 | Lysophosphatidylcholine with acyl residue C16:0 |
| lysoPC a C16:1 | Lysophosphatidylcholine with acyl residue C16:1 |
| lysoPC a C17:0 | Lysophosphatidylcholine with acyl residue C17:0 |
| lysoPC a C18:0 | Lysophosphatidylcholine with acyl residue C18:0 |
| lysoPC a C18:1 | Lysophosphatidylcholine with acyl residue C18:1 |
| lysoPC a C18:2 | Lysophosphatidylcholine with acyl residue C18:2 |
| lysoPC a C20:3 | Lysophosphatidylcholine with acyl residue C20:3 |
| lysoPC a C20:4 | Lysophosphatidylcholine with acyl residue C20:4 |
| lysoPC a C24:0 | Lysophosphatidylcholine with acyl residue C24:0 |
| lysoPC a C26:0 | Lysophosphatidylcholine with acyl residue C26:0 |
| lysoPC a C26:1 | Lysophosphatidylcholine with acyl residue C26:1 |
| lysoPC a C28:0 | Lysophosphatidylcholine with acyl residue C28:0 |
| lysoPC a C28:1 | Lysophosphatidylcholine with acyl residue C28:1 |
| PC aa C24:0 | Phosphatidylcholine with diacyl residue sum C24:0 |
| PC aa C26:0 | Phosphatidylcholine with diacyl residue sum C26:0 |
| PC aa C28:1 | Phosphatidylcholine with diacyl residue sum C28:1 |
| PC aa C30:0 | Phosphatidylcholine with diacyl residue sum C30:0 |
| PC aa C30:2 | Phosphatidylcholine with diacyl residue sum C30:2 |
| PC aa C32:0 | Phosphatidylcholine with diacyl residue sum C32:0 |
| PC aa C32:1 | Phosphatidylcholine with diacyl residue sum C32:1 |
| PC aa C32:2 | Phosphatidylcholine with diacyl residue sum C32:2 |
| PC aa C32:3 | Phosphatidylcholine with diacyl residue sum C32:3 |
| PC aa C34:1 | Phosphatidylcholine with diacyl residue sum C34:1 |
| PC aa C34:2 | Phosphatidylcholine with diacyl residue sum C34:2 |
| PC aa C34:3 | Phosphatidylcholine with diacyl residue sum C34:3 |
| PC aa C34:4 | Phosphatidylcholine with diacyl residue sum C34:4 |
| PC aa C36:0 | Phosphatidylcholine with diacyl residue sum C36:0 |
| PC aa C36:1 | Phosphatidylcholine with diacyl residue sum C36:1 |
| PC aa C36:2 | Phosphatidylcholine with diacyl residue sum C36:2 |
| PC aa C36:3 | Phosphatidylcholine with diacyl residue sum C36:3 |
| PC aa C36:4 | Phosphatidylcholine with diacyl residue sum C36:4 |
| PC aa C36:5 | Phosphatidylcholine with diacyl residue sum C36:5 |
| PC aa C36:6 | Phosphatidylcholine with diacyl residue sum C36:6 |
| PC aa C38:0 | Phosphatidylcholine with diacyl residue sum C38:0 |
| PC aa C38:1 | Phosphatidylcholine with diacyl residue sum C38:1 |
| PC aa C38:3 | Phosphatidylcholine with diacyl residue sum C38:3 |
| PC aa C38:4 | Phosphatidylcholine with diacyl residue sum C38:4 |
| PC aa C38:5 | Phosphatidylcholine with diacyl residue sum C38:5 |
| PC aa C38:6 | Phosphatidylcholine with diacyl residue sum C38:6 |
| PC aa C40:1 | Phosphatidylcholine with diacyl residue sum C40:1 |
| PC aa C40:2 | Phosphatidylcholine with diacyl residue sum C40:2 |
| PC aa C40:3 | Phosphatidylcholine with diacyl residue sum C40:3 |
| PC aa C40:4 | Phosphatidylcholine with diacyl residue sum C40:4 |
| PC aa C40:5 | Phosphatidylcholine with diacyl residue sum C40:5 |
| PC aa C40:6 | Phosphatidylcholine with diacyl residue sum C40:6 |
| PC aa C42:0 | Phosphatidylcholine with diacyl residue sum C42:0 |
| PC aa C42:1 | Phosphatidylcholine with diacyl residue sum C42:1 |
| PC aa C42:2 | Phosphatidylcholine with diacyl residue sum C42:2 |
| PC aa C42:4 | Phosphatidylcholine with diacyl residue sum C42:4 |
| PC aa C42:5 | Phosphatidylcholine with diacyl residue sum C42:5 |
| PC aa C42:6 | Phosphatidylcholine with diacyl residue sum C42:6 |
| PC ae C30:0 | Phosphatidylcholine with acyl-alkyl residue sum C30:0 |
| PC ae C30:1 | Phosphatidylcholine with acyl-alkyl residue sum C30:1 |
| PC ae C30:2 | Phosphatidylcholine with acyl-alkyl residue sum C30:2 |
| PC ae C32:1 | Phosphatidylcholine with acyl-alkyl residue sum C32:1 |
| PC ae C32:2 | Phosphatidylcholine with acyl-alkyl residue sum C32:2 |
| PC ae C34:0 | Phosphatidylcholine with acyl-alkyl residue sum C34:0 |
| PC ae C34:1 | Phosphatidylcholine with acyl-alkyl residue sum C34:1 |
| PC ae C34:2 | Phosphatidylcholine with acyl-alkyl residue sum C34:2 |
| PC ae C34:3 | Phosphatidylcholine with acyl-alkyl residue sum C34:3 |
| PC ae C36:0 | Phosphatidylcholine with acyl-alkyl residue sum C36:0 |
| PC ae C36:1 | Phosphatidylcholine with acyl-alkyl residue sum C36:1 |
| PC ae C36:2 | Phosphatidylcholine with acyl-alkyl residue sum C36:2 |
| PC ae C36:3 | Phosphatidylcholine with acyl-alkyl residue sum C36:3 |
| PC ae C36:4 | Phosphatidylcholine with acyl-alkyl residue sum C36:4 |
| PC ae C36:5 | Phosphatidylcholine with acyl-alkyl residue sum C36:5 |
| PC ae C38:0 | Phosphatidylcholine with acyl-alkyl residue sum C38:0 |
| PC ae C38:1 | Phosphatidylcholine with acyl-alkyl residue sum C38:1 |
| PC ae C38:2 | Phosphatidylcholine with acyl-alkyl residue sum C38:2 |
| PC ae C38:3 | Phosphatidylcholine with acyl-alkyl residue sum C38:3 |
| PC ae C38:4 | Phosphatidylcholine with acyl-alkyl residue sum C38:4 |
| PC ae C38:5 | Phosphatidylcholine with acyl-alkyl residue sum C38:5 |
| PC ae C38:6 | Phosphatidylcholine with acyl-alkyl residue sum C38:6 |
| PC ae C40:1 | Phosphatidylcholine with acyl-alkyl residue sum C40:1 |
| PC ae C40:2 | Phosphatidylcholine with acyl-alkyl residue sum C40:2 |
| PC ae C40:3 | Phosphatidylcholine with acyl-alkyl residue sum C40:3 |
| PC ae C40:4 | Phosphatidylcholine with acyl-alkyl residue sum C40:4 |
| PC ae C40:5 | Phosphatidylcholine with acyl-alkyl residue sum C40:5 |
| PC ae C40:6 | Phosphatidylcholine with acyl-alkyl residue sum C40:6 |
| PC ae C42:0 | Phosphatidylcholine with acyl-alkyl residue sum C42:0 |
| PC ae C42:1 | Phosphatidylcholine with acyl-alkyl residue sum C42:1 |
| PC ae C42:2 | Phosphatidylcholine with acyl-alkyl residue sum C42:2 |
| PC ae C42:3 | Phosphatidylcholine with acyl-alkyl residue sum C42:3 |
| PC ae C42:4 | Phosphatidylcholine with acyl-alkyl residue sum C42:4 |
| PC ae C42:5 | Phosphatidylcholine with acyl-alkyl residue sum C42:5 |
| PC ae C44:3 | Phosphatidylcholine with acyl-alkyl residue sum C44:3 |
| PC ae C44:4 | Phosphatidylcholine with acyl-alkyl residue sum C44:4 |
| PC ae C44:5 | Phosphatidylcholine with acyl-alkyl residue sum C44:5 |
| PC ae C44:6 | Phosphatidylcholine with acyl-alkyl residue sum C44:6 |

### Table 2. List of metabolites measured in Biocrates p400 kit:

**Table 2a. Amino Acids**

| **Abbreviation** | **Analyte** |
|---|---|
| Ala | Alanine |
| Arg | Arginine |
| Asn | Asparagine |
| Asp | Aspartate |
| Cit | Citrulline |
| Gln | Glutamine |
| Glu | Glutamate |
| Gly | Glycine |
| His | Histidine |
| Ile | Isoleucine |
| xLeu | Leucine + Isoleucine |
| Lys | Lysine |
| Met | Methionine |
| Orn | Ornithine |
| Phe | Phenylalanine |
| Pro | Proline |
| Ser | Serine |
| Thr | Threonine |
| Trp | Tryptophan |
| Tyr | Tyrosine |
| Val | Valine |

**Table 2b. Biogenic Amines**

| **Abbreviation** | **Analyte** |
|---|---|
| AcOrn | Acetylornithine |
| ADMA | Asymmetric dimethylarginine |
| alpha-AAA | alpha-Aminoadipic acid |
| Carnosine | Carnosine |
| c4-OH-Pro | *cis*-4-Hydroxyproline |
| Creatinine | Creatinine |
| DOPA | dihydroxyphenylalanine |
| Dopamine | Dopamine |
| Histamine | Histamine |
| Kynurenine | Kynurenine |
| Met-SO | Methionine-sulfoxide |
| Nitro-Tyr | Nitro-tyrosine |
| PEA | Phenylethylamine |
| Putrescine | Putrescine |
| Sarcosine | Sarcosine |
| SDMA | Symmetric dimethylarginine |
| Serotonin | Serotonin |
| Spermidine | Spermidine |
| Spermine | Spermine |
| Taurine | Taurine |
| t4-OH-Pro | *trans*-4-Hydroxyproline |

**Table 2c. Monosaccharides**

| **Abbreviation** | **Analyte** |
|---|---|
| H1 | Hexoses (including glucose) |

**Table 2d. Acylcarnitines**

| **Abbreviation** | **Analyte** |
|---|---|
| AC(0:0) | Carnitine |
| AC(2:0) | Acetylcarnitine |
| AC(3:0) | Propionylcarnitine |
| AC(3:0-DC) | Malonylcarnitine |
| AC(3:0-OH) | Hydroxypropionylcarnitine |
| AC(3:1) | Propenoylcarnitine |
| AC(4:0) | Butyrylcarnitine |
| AC(4:0-DC) | Methylmalonylcamitine |
| AC(4:0-OH) | Hydroxybutyrylcarnitine |
| AC(4:1) | Butenylcamitine |
| AC(4:1-DC) | Fumarylcarnitine |
| AC(5:0) | Valerylcamitine |
| AC(5:0-DC) | Glutarylcarnitine |
| AC(5:0-OH) | Hydroxyvalerylcarnitine |
| AC(5:1) | Tiglylcarnitine |
| AC(5:1-DC) | Glutaconylcamitine |
| AC(6:0) | Hexanoylcarnitine |
| AC(6:0-DC) | Adipoylcarnitine |
| AC(6:0-OH) | Hydroxyhexanoylcarnitine |
| AC(6:1) | Hexenoylcarnitine |
| AC(7:0) | Heptanoylcarnitine |
| AC(7:0-DC) | Pimelylcarnitine |
| AC(8:0) | Octanoylcarnitine |
| AC(8:1) | Octenoylcarnitine |
| AC(8:1-OH) | Hydroxyoctenoylcarnitine |
| AC(9:0) | Nonaylcarnitine |
| AC(10:0) | Decanoylcarnitine |
| AC(10:1) | Decenoylcarnitine |
| AC(10:2) | Decadienylcarnitine |
| AC(10:3) | Decatrienoylcarnitine |
| AC(11:0) | Dimethylnonanoylcamitine |
| AC(12:0) | Dodecanoylcarnitine |
| AC(12:0-DC) | Dodecanedioylcarnitine |
| AC(12:1) | Dodecenoylcarnitine |
| AC(13:0) | Tridecanoylcarnitine |
| AC(14:0) | Tetradecanoylcarnitine |
| AC(14:0-OH) | Hydroxytetradecenoylcarnitine |
| AC(14:1) | Tetradecenoylcarnitine |
| AC(14:1-DC) | Carboxytridecenoylcarnitine |
| AC(14:1-OH) | Hydroxytetradecenoylcarnitine |
| AC(14:2) | Tetradecadienylcarnitine |
| AC(14:2-OH) | Hydroxytetradecadienylcarnitine |
| AC(15:0) | Pentadecanoylcarnitine |
| AC(16:0) | Hexadecanoylcarnitine |
| AC(16:0-OH) | Hydroxyhexadecanoylcarnitine |
| AC(16:1) | Hexadecenoylcarnitine |
| AC(16:1-OH) | Hydroxyhexadecenoylcarnitine |
| AC(16:2) | Hexadecadienylcarnitine |
| AC(16:2-OH) | Hydroxyhexadecadienylcarnitine |
| AC(17:0) | Heptadecanoylcarnitine |
| AC(18:0) | Octadecanoylcarnitine |
| AC(18:1) | Octadecenoylcarnitine |
| AC(18:1-OH) | Hydroxyoctadecenoylcarnitine |
| AC(18:2) | Octadecadienylcarnitine |
| AC(19:0) | Nonadecanoylcarnitine |

**Table 2e. Diglycerides**

| **Abbreviation** | **Analyte** |
|---|---|
| DG(32:1) | Diglyceride with diacyl residue sum C32:1 |
| DG(32:2) | Diglyceride with diacyl residue sum C32:2 |
| DG(34:1) | Diglyceride with diacyl residue sum C34:1 |
| DG(34:3) | Diglyceride with diacyl residue sum C34:3 |
| DG(36:2) | Diglyceride with diacyl residue sum C36:2 |
| DG(36:3) | Diglyceride with diacyl residue sum C36:3 |
| DG(36:4) | Diglyceride with diacyl residue sum C36:4 |
| DG(38:0) | Diglyceride with diacyl residue sum C38:0 |
| DG(38:5) | Diglyceride with diacyl residue sum C38:5 |
| DG(39:0) | Diglyceride with diacyl residue sum C39:0 |
| DG(41:1) | Diglyceride with diacyl residue sum C41:1 |
| DG(42:0) | Diglyceride with diacyl residue sum C42:0 |
| DG(42:1) | Diglyceride with diacyl residue sum C42:1 |
| DG(42:2) | Diglyceride with diacyl residue sum C42:2 |
| DG(44:3) | Diglyceride with diacyl residue sum C44:3 |
| DG-O(32:2) | Diglyceride with acyl-alkyl residue sum C32:2 |
| DG-O(34:1) | Diglyceride with acyl-alkyl residue sum C34:1 |
| DG-O(36:4) | Diglyceride with acyl-alkyl residue sum C36:4 |

**Table 2f. Triglycerides**

| **Abbreviation** | **Analyte** |
|---|---|
| TG(44:1) | Triglyceride with triacyl residue sum C44:1 |
| TG(44:2) | Triglyceride with triacyl residue sum C44:2 |
| TG(44:4) | Triglyceride with triacyl residue sum C44:4 |
| TG(46:2) | Triglyceride with triacyl residue sum C46:2 |
| TG(48:1) | Triglyceride with triacyl residue sum C48:1 |
| TG(48:2) | Triglyceride with triacyl residue sum C48:2 |
| TG(48:3) | Triglyceride with triacyl residue sum C48:3 |
| TG(49:1) | Triglyceride with triacyl residue sum C49:1 |
| TG(49:2) | Triglyceride with triacyl residue sum C49:2 |
| TG(50:1) | Triglyceride with triacyl residue sum C50:1 |
| TG(50:2) | Triglyceride with triacyl residue sum C50:2 |
| TG(50:3) | Triglyceride with triacyl residue sum C50:3 |
| TG(50:4) | Triglyceride with triacyl residue sum C50:4 |
| TG(51:1) | Triglyceride with triacyl residue sum C51:1 |
| TG(51:2) | Triglyceride with triacyl residue sum C51:2 |
| TG(51:3) | Triglyceride with triacyl residue sum C51:3 |
| TG(51:4) | Triglyceride with triacyl residue sum C51:4 |
| TG(51:5) | Triglyceride with triacyl residue sum C51:5 |
| TG(52:2) | Triglyceride with triacyl residue sum C52:2 |
| TG(52:3) | Triglyceride with triacyl residue sum C52:3 |
| TG(52:4) | Triglyceride with triacyl residue sum C52:4 |
| TG(52:5) | Triglyceride with triacyl residue sum C52:5 |
| TG(52:6) | Triglyceride with triacyl residue sum C52:6 |
| TG(52:7) | Triglyceride with triacyl residue sum C52:7 |
| TG(53:3) | Triglyceride with triacyl residue sum C53:3 |
| TG(53:4) | Triglyceride with triacyl residue sum C53:4 |
| TG(53:5) | Triglyceride with triacyl residue sum C53:5 |
| TG(53:6) | Triglyceride with triacyl residue sum C53:6 |
| TG(54:2) | Triglyceride with triacyl residue sum C54:2 |
| TG(54:3) | Triglyceride with triacyl residue sum C54:3 |
| TG(54:4) | Triglyceride with triacyl residue sum C54:4 |
| TG(54:5) | Triglyceride with triacyl residue sum C54:5 |
| TG(54:6) | Triglyceride with triacyl residue sum C54:6 |
| TG(54:7) | Triglyceride with triacyl residue sum C54:7 |
| TG(55:6) | Triglyceride with triacyl residue sum C55:6 |
| TG(55:7) | Triglyceride with triacyl residue sum C55:7 |
| TG(55:8) | Triglyceride with triacyl residue sum C55:8 |
| TG(55:9) | Triglyceride with triacyl residue sum C55:9 |
| TG(56:6) | Triglyceride with triacyl residue sum C56:6 |
| TG(56:7) | Triglyceride with triacyl residue sum C56:7 |
| TG(56:8) | Triglyceride with triacyl residue sum C56:8 |
| TG(56:9) | Triglyceride with triacyl residue sum C56:9 |

**Table 2g. Lysophosphatidylcholines**

| **Abbreviation** | **Analyte** |
|---|---|
| LPC(12:0) | Lysophosphatidylcholine with acyl residue C12:0 |
| LPC(14:0) | Lysophosphatidylcholine with acyl residue C14:0 |
| LPC(15:0) | Lysophosphatidylcholine with acyl residue C15:0 |
| LPC(16:0) | Lysophosphatidylcholine with acyl residue C16:0 |
| LPC(16:1) | Lysophosphatidylcholine with acyl residue C16:1 |
| LPC(17:0) | Lysophosphatidylcholine with acyl residue C17:0 |
| LPC(17:1) | Lysophosphatidylcholine with acyl residue C17:1 |
| LPC(18:0) | Lysophosphatidylcholine with acyl residue C18:0 |
| LPC(18:1) | Lysophosphatidylcholine with acyl residue C18:1 |
| LPC(18:2) | Lysophosphatidylcholine with acyl residue C18:2 |
| LPC(20:0) | Lysophosphatidylcholine with acyl residue C20:0 |
| LPC(20:1) | Lysophosphatidylcholine with acyl residue C20:1 |
| LPC(20:2) | Lysophosphatidylcholine with acyl residue C20:2 |
| LPC(20:3) | Lysophosphatidylcholine with acyl residue C20:3 |
| LPC(20:4) | Lysophosphatidylcholine with acyl residue C20:4 |
| LPC(22:5) | Lysophosphatidylcholine with acyl residue C22:5 |
| LPC(22:6) | Lysophosphatidylcholine with acyl residue C22:6 |
| LPC(24:0) | Lysophosphatidylcholine with acyl residue C24:0 |
| LPC(24:1) | Lysophosphatidylcholine with acyl residue C24:1 |
| LPC-O(16:1) | Lysophosphatidylcholine with acyl-alkyl residue sum C16:1 |
| LPC-O(17:1) | Lysophosphatidylcholine with acyl-alkyl residue sum C17:1 |
| LPC-O(18:0) | Lysophosphatidylcholine with acyl-alkyl residue sum C18:0 |
| LPC-O(18:1) | Lysophosphatidylcholine with acyl-alkyl residue sum C18:1 |
| LPC-O(18:2) | Lysophosphatidylcholine with acyl-alkyl residue sum C18:2 |

**Table 2h. Phosphatidylcholines**

| **Abbreviation** | **Analyte** |
|---|---|
| PC(24:0) | Phosphatidylcholine with diacyl residue sum C24:0 |
| PC(25:0) | Phosphatidylcholine with diacyl residue sum C25:0 |
| PC(26:0) | Phosphatidylcholine with diacyl residue sum C26:0 |
| PC(27:0) | Phosphatidylcholine with diacyl residue sum C27:0 |
| PC(27:1) | Phosphatidylcholine with diacyl residue sum C27:1 |
| PC(28:1) | Phosphatidylcholine with diacyl residue sum C28:1 |
| PC(29:0) | Phosphatidylcholine with diacyl residue sum C29:0 |
| PC(29:1) | Phosphatidylcholine with diacyl residue sum C29:1 |
| PC(29:2) | Phosphatidylcholine with diacyl residue sum C29:2 |
| PC(30:0) | Phosphatidylcholine with diacyl residue sum C30:0 |
| PC(30:1) | Phosphatidylcholine with diacyl residue sum C30:1 |
| PC(30:2) | Phosphatidylcholine with diacyl residue sum C30:2 |
| PC(30:3) | Phosphatidylcholine with diacyl residue sum C30:3 |
| PC(31:0) | Phosphatidylcholine with diacyl residue sum C31:0 |
| PC(31:1) | Phosphatidylcholine with diacyl residue sum C31:1 |
| PC(31:2) | Phosphatidylcholine with diacyl residue sum C31:2 |
| PC(31:3) | Phosphatidylcholine with diacyl residue sum C31:3 |
| PC(32:0) | Phosphatidylcholine with diacyl residue sum C32:0 |
| PC(32:1) | Phosphatidylcholine with diacyl residue sum C32:1 |
| PC(32:2) | Phosphatidylcholine with diacyl residue sum C32:2 |
| PC(32:3) | Phosphatidylcholine with diacyl residue sum C32:3 |
| PC(32:4) | Phosphatidylcholine with diacyl residue sum C32:4 |
| PC(32:5) | Phosphatidylcholine with diacyl residue sum C32:5 |
| PC(32:6) | Phosphatidylcholine with diacyl residue sum C32:6 |
| PC(33:0) | Phosphatidylcholine with diacyl residue sum C33:0 |
| PC(33:1) | Phosphatidylcholine with diacyl residue sum C33:1 |
| PC(33:2) | Phosphatidylcholine with diacyl residue sum C33:2 |
| PC(33:3) | Phosphatidylcholine with diacyl residue sum C33:3 |
| PC(33:4) | Phosphatidylcholine with diacyl residue sum C33:4 |
| PC(33:5) | Phosphatidylcholine with diacyl residue sum C33:5 |
| PC(34:0) | Phosphatidylcholine with diacyl residue sum C34:0 |
| PC(34:1) | Phosphatidylcholine with diacyl residue sum C34:1 |
| PC(34:2) | Phosphatidylcholine with diacyl residue sum C34:2 |
| PC(34:3) | Phosphatidylcholine with diacyl residue sum C34:3 |
| PC(34:4) | Phosphatidylcholine with diacyl residue sum C34:4 |
| PC(34:5) | Phosphatidylcholine with diacyl residue sum C34:5 |
| PC(35:0) | Phosphatidylcholine with diacyl residue sum C35:0 |
| PC(35:1) | Phosphatidylcholine with diacyl residue sum C35:1 |
| PC(35:2) | Phosphatidylcholine with diacyl residue sum C35:2 |
| PC(35:3) | Phosphatidylcholine with diacyl residue sum C35:3 |
| PC(35:4) | Phosphatidylcholine with diacyl residue sum C35:4 |
| PC(35:5) | Phosphatidylcholine with diacyl residue sum C35:5 |
| PC(36:0) | Phosphatidylcholine with diacyl residue sum C36:0 |
| PC(36:1) | Phosphatidylcholine with diacyl residue sum C36:1 |
| PC(36:2) | Phosphatidylcholine with diacyl residue sum C36:2 |
| PC(36:3) | Phosphatidylcholine with diacyl residue sum C36:3 |
| PC(36:4) | Phosphatidylcholine with diacyl residue sum C36:4 |
| PC(36:5) | Phosphatidylcholine with diacyl residue sum C36:5 |
| PC(36:6) | Phosphatidylcholine with diacyl residue sum C36:6 |
| PC(37:0) | Phosphatidylcholine with diacyl residue sum C37:0 |
| PC(37:1) | Phosphatidylcholine with diacyl residue sum C37:1 |
| PC(37:2) | Phosphatidylcholine with diacyl residue sum C37:2 |
| PC(37:3) | Phosphatidylcholine with diacyl residue sum C37:3 |
| PC(37:4) | Phosphatidylcholine with diacyl residue sum C37:4 |
| PC(37:5) | Phosphatidylcholine with diacyl residue sum C37:5 |
| PC(37:6) | Phosphatidylcholine with diacyl residue sum C37:6 |
| PC(37:7) | Phosphatidylcholine with diacyl residue sum C37:7 |
| PC(38:0) | Phosphatidylcholine with diacyl residue sum C38:0 |
| PC(38:1) | Phosphatidylcholine with diacyl residue sum C38:1 |
| PC(38:2) | Phosphatidylcholine with diacyl residue sum C38:2 |
| PC(38:3) | Phosphatidylcholine with diacyl residue sum C38:3 |
| PC(38:4) | Phosphatidylcholine with diacyl residue sum C38:4 |
| PC(38:5) | Phosphatidylcholine with diacyl residue sum C38:5 |
| PC(38:6) | Phosphatidylcholine with diacyl residue sum C38:6 |
| PC(38:7) | Phosphatidylcholine with diacyl residue sum C38:7 |
| PC(39:0) | Phosphatidylcholine with diacyl residue sum C39:0 |
| PC(39:1) | Phosphatidylcholine with diacyl residue sum C39:1 |
| PC(39:2) | Phosphatidylcholine with diacyl residue sum C39:2 |
| PC(39:3) | Phosphatidylcholine with diacyl residue sum C39:3 |
| PC(39:4) | Phosphatidylcholine with diacyl residue sum C39:4 |
| PC(39:5) | Phosphatidylcholine with diacyl residue sum C39:5 |
| PC(39:6) | Phosphatidylcholine with diacyl residue sum C39:6 |
| PC(39:7) | Phosphatidylcholine with diacyl residue sum C39:7 |
| PC(40:1) | Phosphatidylcholine with diacyl residue sum C40:1 |
| PC(40:2) | Phosphatidylcholine with diacyl residue sum C40:2 |
| PC(40:3) | Phosphatidylcholine with diacyl residue sum C40:3 |
| PC(40:4) | Phosphatidylcholine with diacyl residue sum C40:4 |
| PC(40:5) | Phosphatidylcholine with diacyl residue sum C40:5 |
| PC(40:6) | Phosphatidylcholine with diacyl residue sum C40:6 |
| PC(40:7) | Phosphatidylcholine with diacyl residue sum C40:7 |
| PC(40:8) | Phosphatidylcholine with diacyl residue sum C40:8 |
| PC(40:9) | Phosphatidylcholine with diacyl residue sum C40:9 |
| PC(41:1) | Phosphatidylcholine with diacyl residue sum C41:1 |
| PC(41:2) | Phosphatidylcholine with diacyl residue sum C41:2 |
| PC(41:3) | Phosphatidylcholine with diacyl residue sum C41:3 |
| PC(41:4) | Phosphatidylcholine with diacyl residue sum C41:4 |
| PC(41:5) | Phosphatidylcholine with diacyl residue sum C41:5 |
| PC(41:8) | Phosphatidylcholine with diacyl residue sum C41:8 |
| PC(42:0) | Phosphatidylcholine with diacyl residue sum C42:0 |
| PC(42:1) | Phosphatidylcholine with diacyl residue sum C42:1 |
| PC(42:2) | Phosphatidylcholine with diacyl residue sum C42:2 |
| PC(42:3) | Phosphatidylcholine with diacyl residue sum C42:3 |
| PC(42:4) | Phosphatidylcholine with diacyl residue sum C42:4 |
| PC(42:5) | Phosphatidylcholine with diacyl residue sum C42:5 |
| PC(42:6) | Phosphatidylcholine with diacyl residue sum C42:6 |
| PC(42:7) | Phosphatidylcholine with diacyl residue sum C42:7 |
| PC(42:10) | Phosphatidylcholine with diacyl residue sum C42:10 |
| PC(43:2) | Phosphatidylcholine with diacyl residue sum C43:2 |
| PC(43:6) | Phosphatidylcholine with diacyl residue sum C43:6 |
| PC(44:1) | Phosphatidylcholine with diacyl residue sum C44:1 |
| PC(44:3) | Phosphatidylcholine with diacyl residue sum C44:3 |
| PC(44:5) | Phosphatidylcholine with diacyl residue sum C44:5 |
| PC(44:6) | Phosphatidylcholine with diacyl residue sum C44:6 |
| PC(44:7) | Phosphatidylcholine with diacyl residue sum C44:7 |
| PC(44:10) | Phosphatidylcholine with diacyl residue sum C44:10 |
| PC(44:12) | Phosphatidylcholine with diacyl residue sum C44:12 |
| PC(46:1) | Phosphatidylcholine with diacyl residue sum C46:1 |
| PC(46:2) | Phosphatidylcholine with diacyl residue sum C46:2 |
| PC-0(26:0) | Phosphatidylcholine with acyl-alkyl residue sum C26:0 |
| PC-O(26:1) | Phosphatidylcholine with acyl-alkyl residue sum C26:1 |
| PC-O(28:0) | Phosphatidylcholine with acyl-alkyl residue sum C28:0 |
| PC-O(28:1) | Phosphatidylcholine with acyl-alkyl residue sum C28:1 |
| PC-O(29:0) | Phosphatidylcholine with acyl-alkyl residue sum C29:0 |
| PC-O(30:0) | Phosphatidylcholine with acyl-alkyl residue sum C30:0 |
| PC-O(30:1) | Phosphatidylcholine with acyl-alkyl residue sum C30:1 |
| PC-O(30:2) | Phosphatidylcholine with acyl-alkyl residue sum C30:2 |
| PC-O(31:0) | Phosphatidylcholine with acyl-alkyl residue sum C31:0 |
| PC-O(31:1) | Phosphatidylcholine with acyl-alkyl residue sum C31:1 |
| PC-O(31:3) | Phosphatidylcholine with acyl-alkyl residue sum C31:3 |
| PC-O(32:0) | Phosphatidylcholine with acyl-alkyl residue sum C32:0 |
| PC-O(32:1) | Phosphatidylcholine with acyl-alkyl residue sum C32:1 |
| PC-O(32:2) | Phosphatidylcholine with acyl-alkyl residue sum C32:2 |
| PC-O(32:3) | Phosphatidylcholine with acyl-alkyl residue sum C32:3 |
| PC-O(33:0) | Phosphatidylcholine with acyl-alkyl residue sum C33:0 |
| PC-O(33:1) | Phosphatidylcholine with acyl-alkyl residue sum C33:1 |
| PC-O(33:2) | Phosphatidylcholine with acyl-alkyl residue sum C33:2 |
| PC-O(33:3) | Phosphatidylcholine with acyl-alkyl residue sum C33:3 |
| PC-O(33:4) | Phosphatidylcholine with acyl-alkyl residue sum C33:4 |
| PC-O(33:6) | Phosphatidylcholine with acyl-alkyl residue sum C33:6 |
| PC-O(34:0) | Phosphatidylcholine with acyl-alkyl residue sum C34:0 |
| PC-O(34:1) | Phosphatidylcholine with acyl-alkyl residue sum C34:1 |
| PC-O(34:2) | Phosphatidylcholine with acyl-alkyl residue sum C34:2 |
| PC-O(34:3) | Phosphatidylcholine with acyl-alkyl residue sum C34:3 |
| PC-O(34:4) | Phosphatidylcholine with acyl-alkyl residue sum C34:4 |
| PC-O(35:3) | Phosphatidylcholine with acyl-alkyl residue sum C35:3 |
| PC-O(35:4) | Phosphatidylcholine with acyl-alkyl residue sum C35:4 |
| PC-O(36:0) | Phosphatidylcholine with acyl-alkyl residue sum C36:0 |
| PC-O(36:1) | Phosphatidylcholine with acyl-alkyl residue sum C36:1 |
| PC-O(36:2) | Phosphatidylcholine with acyl-alkyl residue sum C36:2 |
| PC-O(36:3) | Phosphatidylcholine with acyl-alkyl residue sum C36:3 |
| PC-O(36:4) | Phosphatidylcholine with acyl-alkyl residue sum C36:4 |
| PC-O(36:5) | Phosphatidylcholine with acyl-alkyl residue sum C36:5 |
| PC-O(36:6) | Phosphatidylcholine with acyl-alkyl residue sum C36:6 |
| PC-O(37:6) | Phosphatidylcholine with acyl-alkyl residue sum C37:6 |
| PC-O(37:7) | Phosphatidylcholine with acyl-alkyl residue sum C37:7 |
| PC-O(38:0) | Phosphatidylcholine with acyl-alkyl residue sum C38:0 |
| PC-O(38:1) | Phosphatidylcholine with acyl-alkyl residue sum C38:1 |
| PC-O(38:2) | Phosphatidylcholine with acyl-alkyl residue sum C38:2 |
| PC-O(38:3) | Phosphatidylcholine with acyl-alkyl residue sum C38:3 |
| PC-O(38:4) | Phosphatidylcholine with acyl-alkyl residue sum C38:4 |
| PC-O(38:5) | Phosphatidylcholine with acyl-alkyl residue sum C38:5 |
| PC-O(38:6) | Phosphatidylcholine with acyl-alkyl residue sum C38:6 |
| PC-O(40:0) | Phosphatidylcholine with acyl-alkyl residue sum C40:0 |
| PC-O(40:1) | Phosphatidylcholine with acyl-alkyl residue sum C40:1 |
| PC-O(40:2) | Phosphatidylcholine with acyl-alkyl residue sum C40:2 |
| PC-O(40:3) | Phosphatidylcholine with acyl-alkyl residue sum C40:3 |
| PC-O(40:4) | Phosphatidylcholine with acyl-alkyl residue sum C40:4 |
| PC-O(40:5) | Phosphatidylcholine with acyl-alkyl residue sum C40:5 |
| PC-O(40:6) | Phosphatidylcholine with acyl-alkyl residue sum C40:6 |
| PC-O(40:7) | Phosphatidylcholine with acyl-alkyl residue sum C40:7 |
| PC-O(40:8) | Phosphatidylcholine with acyl-alkyl residue sum C40:8 |
| PC-O(42:0) | Phosphatidylcholine with acyl-alkyl residue sum C42:0 |
| PC-O(42:1) | Phosphatidylcholine with acyl-alkyl residue sum C42:1 |
| PC-O(42:2) | Phosphatidylcholine with acyl-alkyl residue sum C42:2 |
| PC-O(42:3) | Phosphatidylcholine with acyl-alkyl residue sum C42:3 |
| PC-O(42:4) | Phosphatidylcholine with acyl-alkyl residue sum C42:4 |
| PC-O(42:5) | Phosphatidylcholine with acyl-alkyl residue sum C42:5 |
| PC-O(42:6) | Phosphatidylcholine with acyl-alkyl residue sum C42:6 |
| PC-O(44:3) | Phosphatidylcholine with acyl-alkyl residue sum C44:3 |
| PC-O(44:4) | Phosphatidylcholine with acyl-alkyl residue sum C44:4 |
| PC-O(44:5) | Phosphatidylcholine with acyl-alkyl residue sum C44:5 |
| PC-O(44:6) | Phosphatidylcholine with acyl-alkyl residue sum C44:6 |

**Table 2i. Sphingomyelins**

| **Abbreviation** | **Analyte** |
|---|---|
| SM(30:1) | Sphingomyelin with acyl residue sum C30:1 |
| SM(31:0) | Sphingomyelin with acyl residue sum C31:0 |
| SM(31:1) | Sphingomyelin with acyl residue sum C31:1 |
| SM(32:1) | Sphingomyelin with acyl residue sum C32:1 |
| SM(32:2) | Sphingomyelin with acyl residue sum C32:2 |
| SM(33:1) | Sphingomyelin with acyl residue sum C33:1 |
| SM(33:2) | Sphingomyelin with acyl residue sum C33:2 |
| SM(34:1) | Sphingomyelin with acyl residue sum C34:1 |
| SM(34:2) | Sphingomyelin with acyl residue sum C34:2 |
| SM(35:1) | Sphingomyelin with acyl residue sum C35:1 |
| SM(36:0) | Sphingomyelin with acyl residue sum C36:0 |
| SM(36:1) | Sphingomyelin with acyl residue sum C36:1 |
| SM(36:2) | Sphingomyelin with acyl residue sum C36:2 |
| SM(37:1) | Sphingomyelin with acyl residue sum C37:1 |
| SM(38:1) | Sphingomyelin with acyl residue sum C38:1 |
| SM(38:2) | Sphingomyelin with acyl residue sum C38:2 |
| SM(38:3) | Sphingomyelin with acyl residue sum C38:3 |
| SM(39:1) | Sphingomyelin with acyl residue sum C39:1 |
| SM(39:2) | Sphingomyelin with acyl residue sum C39:2 |
| SM(40:1) | Sphingomyelin with acyl residue sum C40:1 |
| SM(40:2) | Sphingomyelin with acyl residue sum C40:2 |
| SM(40:4) | Sphingomyelin with acyl residue sum C40:4 |
| SM(41:1) | Sphingomyelin with acyl residue sum C41:1 |
| SM(41:2) | Sphingomyelin with acyl residue sum C41:2 |
| SM(42:1) | Sphingomyelin with acyl residue sum C42:1 |
| SM(42:2) | Sphingomyelin with acyl residue sum C42:2 |
| SM(42:3) | Sphingomyelin with acyl residue sum C42:3 |
| SM(43:1) | Sphingomyelin with acyl residue sum C43:1 |
| SM(43:2) | Sphingomyelin with acyl residue sum C43:2 |
| SM(44:1) | Sphingomyelin with acyl residue sum C44:1 |
| SM(44:2) | Sphingomyelin with acyl residue sum C44:2 |

**Table 2j. Ceramides**

| **Abbreviation** | **Analyte** |
|---|---|
| Cer(34:0) | Ceramide (34:0) |
| Cer(34:1) | Ceramide (34:1) |
| Cer(38:1) | Ceramide (38:1) |
| Cer(40:1) | Ceramide (40:1) |
| Cer(41:1) | Ceramide (41:1) |
| Cer(42:1) | Ceramide (42:1) |
| Cer(42:2) | Ceramide (42:2) |
| Cer(43:1) | Ceramide (43:1) |
| Cer(44:0) | Ceramide (44:0) |

**Table 2k. Cholesteryl Esters**

| **Abbreviation** | **Analyte** |
|---|---|
| CE(16:0) | cholesteryl palmitate |
| CE(16:1) | cholesteryl palmitoleate |
| CE(17:0) | cholesteryl heptadecanoate |
| CE(17:1) | cholesteryl heptadecenoate |
| CE(17:2) | 17:2 Cholesterol ester |
| CE(18:1) | Cholesterol vaccenoate |
| CE(18:2) | Cholesterol linoleoate |
| CE(18:3) | Cholesterol linolenoate |
| CE(19:2) | 19:2 Cholesterol ester |
| CE(19:3) | 19:3 Cholesterol ester |
| CE(20:4) | Cholesterol eicsoate |
| CE(20:5) | Cholesteryl eicosapentaenoate |
| CE(22:5) | Cholesterol osbondoate |
| CE(22:6) | Cholesteryl docosahexaenoate |

### REFERENCES

Berghuis, A.M.S., et al., Detecting Blood-Based Biomarkers in Metastatic Breast Cancer: A Systematic Review of Their Current Status and Clinical Utility. International Journal of Molecular Sciences, 2017. 18(2).
Carney, P.A., et al., Discovery of breast cancers within 1 year of a normal screening mammogram: How are they found? Annals of Family Medicine, 2006. 4(6): p. 512-518
Denkert, C., et al., Mass spectrometry-based metabolic profiling reveals different metabolite patterns in invasive ovarian carcinomas and ovarian borderline tumors. Cancer Res, 2006. 66(22): p. 10795-804.
Diaz, L.A., Jr. and A. Bardelli, Liquid biopsies: genotyping circulating tumor DNA. J Clin Oncol, 2014. 32(6): p. 579-86.
Duffy, M.J., et al., Clinical use of biomarkers in breast cancer: Updated guidelines from the European Group on Tumor Markers (EGTM). European Journal of Cancer, 2017. 75: p. 284-298.
Fan, Y., et al., Human plasma metabolomics for identifying differential metabolites and predicting molecular subtypes of breast cancer. Oncotarget, 2016. 7(9): p. 9925-38.
Gunther, U.L., Metabolomics Biomarkers for Breast Cancer. Pathobiology, 2015. 82(3-4): p. 153-65.
Hadi, N.I., et al., Serum Metabolomic Profiles for Breast Cancer Diagnosis, Grading and Staging by Gas Chromatography Mass Spectrometry. Sci Rep, 2017. 7(1): p. 1715.
Harris, L., et al., American society of clinical oncology 2007 update of recommendations for the use of tumor markers in breast cancer. Journal of Clinical Oncology, 2007. 25(33): p. 5287-5312.
Hart, C.D., et al., Metabolomics in Breast Cancer: Current Status and Perspectives. Adv Exp Med Biol, 2016. 882: p. 217-34.
Hsu, P.P. and D.M. Sabatini, Cancer cell metabolism: Warburg and beyond. Cell, 2008. 134(5): p. 703-7.
Janni, W.J., et al., Pooled Analysis of the Prognostic Relevance of Circulating Tumor Cells in Primary Breast Cancer. Clin Cancer Res, 2016. 22(10): p. 2583-93.
Koppenol, W.H., P.L. Bounds, and C.V. Dang, Otto Warburg's contributions to current concepts of cancer metabolism. Nat Rev Cancer, 2011. 11(5): p. 325-37.
Kuhn, T., et al., Higher plasma levels of lysophosphatidylcholine 18:0 are related to a lower risk of common cancers in a prospective metabolomics study. BMC Med, 2016. 14: p. 13.
Locasale, J.W., et al., Phosphoglycerate dehydrogenase diverts glycolytic flux and contributes to oncogenesis. Nat Genet, 2011. 43(9): p. 869-74.
Overman, M.J., et al., Use of Research Biopsies in Clinical Trials: Are Risks and Benefits Adequately Discussed? Journal of Clinical Oncology, 2013. 31(1): p. 17-22.
Perez, E.A., Breast cancer management: opportunities and barriers to an individualized approach. Oncologist, 2011. 16 Suppl 1: p. 20-2.
Redig, A.J. and S. S. McAllister, Breast cancer as a systemic disease: a view of metastasis. Journal of Internal Medicine, 2013. 274(2): p. 113-126.
Siegel, R.L., K.D. Miller, and A. Jemal, Cancer statistics, 2015. CA Cancer J Clin, 2015. 65(1): p. 5-29.
Tenori, L., et al., Serum metabolomic profiles evaluated after surgery may identify patients with oestrogen receptor negative early breast cancer at increased risk of disease recurrence. Results from a retrospective study. Molecular Oncology, 2015. 9(1): p. 128-139.
Tiryakioglu, N.O., et al., miR-19b-3p and miR-4687-5p as novel circulating miRNAs as potential prognostic biomarkers in breast cancer. Cancer Research, 2017. 77.
Torre, L.A., et al., Global cancer statistics, 2012. CA Cancer J Clin, 2015. 65(2): p. 87-108.
Weigelt, B., J.L. Peterse, and L.J. van't Veer, Breast cancer metastasis: Markers and models. Nature Reviews Cancer, 2005. 5(8): p. 591-602.
Zhou, J., Metabonomics studies on serum and urine of patients with breast cancer using 1H-NMR spectroscopy. Oncotarget, 2017.

## Claims

1. Use of a combination of metabolites contained in a blood sample of a mammalian subject, the combination of metabolites comprising
at least the amino acids ornithine (Orn), threonine (Thr), and tryptophan (Trp),
and
at least one acylcarnitine, which is acetylcarnitine (C2 or AC.2.0), in the *in vitro* or *ex vivo* diagnosis of primary breast cancer comprising the detection of the presence of the metabolites in said sample and comparing it to a control sample.

2. The use according to claim 1, comprising further amino acids selected from glutamate (Glu), aspartate (Asp), and histidine (His).

3. The use according to claim 1 or 2, comprising further acylcarnitines selected from propionylcarnitine (C3), hexanoylcarnitine (AC.6.0), dodecanoylcarnitine (AC. 12.0), and hexadecenoylcarnitine (AC.16.1).

4. The use according to any one of claims 1 to 3, furthermore comprising at least one biogenic amine,
preferably methionine sulfoxide (Met-SO).

5. The use according to any one of claims 1 to 4, wherein the blood sample is whole blood, plasma or serum.

6. The use according to any one of claims 1 to 5, wherein the mammalian subject is human.

7. The use according to any one of claims 1 to 6, wherein the metabolites are measured based on a quantitative analytical method,
preferably chromatography, spectroscopy, and mass analyzers/spectrometry.

8. The use according to any one of claims 1 to 6, wherein the metabolites are measured using an antibody-based method, such as an ELISA.

9. The use according to claim 7, wherein chromatography comprises GC, CE, LC, HPLC, and UHPLC.

10. The use according to claim 7, wherein spectroscopy comprises UV/Vis, IR, NIR and NMR.

11. The use according to claim 7, wherein mass analyzers/spectrometry comprises ESI, Quadrupole Mass Analyzers, Ion Trap Mass Analyzers, TOF (Time of Flight) Mass Analyzer, Orbitrap mass analyzer, Magnetic Sector Mass Analyzer, Electrostatic Sector Mass Analyzer, Ion Cyclotron Resonance (ICR) and combinations thereof,
including single quadrupole (Q) and triple quadrupole (QqQ), QqTOF, TOF-TOF, Q-Orbitrap, APCI-QqQ, APCI-QqTOF, MALDI-QqQ, MALDI- QqTOF, and MALDI-TOF-TOF.

12. An *in vitro* or *ex vivo* method for the diagnosis of primary breast cancer, comprising the following steps:
(a) providing a blood sample of a mammalian subject,
(b) determining the amount of a combination of metabolites, the combination of metabolites being as defined in any one of claims 1 to 4, and
(c) comparing said amount determined in (b) with a control sample.

13. The method of claim 12, wherein the blood sample is whole blood, plasma or serum; and/or wherein the mammalian subject is human.

14. The method of claim 12 or 13, wherein the metabolites are measured based on a quantitative analytical method,
preferably chromatography, spectroscopy, and mass analyzers/ spectrometry,
wherein chromatography preferably comprises GC, CE, LC, HPLC, and UHPLC;
wherein spectroscopy preferably comprises UV/Vis, IR, NIR and NMR; and
wherein mass analyzers/spectrometry preferably comprises ESI, Quadrupole Mass Analyzers, Ion Trap Mass Analyzers, TOF (Time of Flight) Mass Analyzer, Orbitrap mass analyzer, Magnetic Sector Mass Analyzer, Electrostatic Sector Mass Analyzer, Ion Cyclotron Resonance (ICR) and combinations thereof,
including single quadrupole (Q) and triple quadrupole (QqQ), QqTOF, TOF-TOF, Q-Orbitrap, APCI-QqQ, APCI-QqTOF, MALDI-QqQ, MALDI- QqTOF, and MALDI-TOF-TOF.

## Patentansprüche

1. Verwendung einer Kombination von Metaboliten, die in einer Blutprobe eines Säugetiers enthalten sind, wobei die Kombination von Metaboliten umfasst
mindestens die Aminosäuren Ornithin (Orn), Threonin (Thr), und Tryptophan (Trp) und
mindestens ein Acylcarnitin, welches Acetylcarnitin (C2 oder AC.2.0) ist,
für die *in vitro* oder *ex vivo* Diagnose von primärem Brustkrebs,
umfassend die Detektion der Anwesenheit der Metaboliten in der Probe und Vergleichen dessen mit einer Kontrollprobe.

2. Die Verwendung gemäß Anspruch 1, umfassend weitere Aminosäuren, die aus Glutamat (Glu), Aspartat (Asp) und Histidin (His) ausgewählt sind.

3. Die Verwendung gemäß Anspruch 1 oder 2, umfassend weitere Acylcarnitine, die aus Propionylcarnitin (C3), Hexanoylcarnitin (AC.6.0), Dodecanoylcarnitin (AC.12.0) und Hexadecenoylcarnitin (AC.16.1) ausgewählt sind.

4. Die Verwendung gemäß einem der Ansprüche 1 bis 3, weiterhin umfassend mindestens ein biogenes Amin,
bevorzugt Methioninsulfoxid (Met-SO).

5. Die Verwendung gemäß einem der Ansprüche 1 bis 4, wobei die Blutprobe Vollblut, Plasma oder Serum ist.

6. Die Verwendung gemäß einem der Ansprüche 1 bis 5, wobei das Säugetier ein Mensch ist.

7. Die Verwendung gemäß einem der Ansprüche 1 bis 6, wobei die Metaboliten basierend auf einem quantitativen analytischen Verfahren gemessen werden,
bevorzugt Chromatographie, Spektroskopie und Massenanalysator/-Spektrometrie.

8. Die Verwendung gemäß einem der Ansprüche 1 bis 6, wobei die Metaboliten unter Verwendung eines Antikörper-basierten Verfahrens gemessen werden, wie beispielsweise ein ELISA.

9. Die Verwendung gemäß Anspruch 7, wobei Chromatographie GC, CE, LC, HPLC und UHPLC umfasst.

10. Die Verwendung gemäß Anspruch 7, wobei Spektroskopie UV/Vis, IR, NIR und NMR umfasst.

11. Die Verwendung gemäß Anspruch 7, wobei Massenanalysator/-Spektrometrie ESI, Quadrupol- Massenanalysator, Ionenfallen- Massenanalysator, TOF (Flugzeit/Time of Flight)- Massenanalysator, Orbitrap- Massenanalysator, Magnetsektor- Massenanalysator, elektrostatischen Sektor- Massenanalysator, Ionen-Zyklotron-Resonanz (ICR) und Kombinationen davon umfasst,
einschließlich Single Quadrupol (Q) und Triple Quadrupol (QqQ), QqTOF, TOF-TOF, Q-Orbitrap, APCI-QqQ, APCI-QqTOF, MALDI-QqQ, MALDI- QqTOF und MALDI-TOF-TOF.

12. Ein *in vitro* oder *ex vivo* Verfahren für die Diagnose von primärem Brustkrebs, umfassend die folgenden Schritte:
(a) Bereitstellen einer Blutprobe eines Säugetiers,
(b) Bestimmen der Menge einer Kombination von Metaboliten, wobei die Kombination von Metaboliten wie in einem der Ansprüche 1 bis 4 definiert ist, und
(c) Vergleichen der in (b) bestimmten Menge mit einer Kontrollprobe.

13. Das Verfahren gemäß Anspruch 12, wobei die Blutprobe Vollblut, Plasma oder Serum ist;
und/oder wobei das Säugetier ein Mensch ist.

14. Das Verfahren gemäß Anspruch 12 oder 13, wobei die Metaboliten basierend auf einem quantitativen analytischen Verfahren gemessen werden,
bevorzugt Chromatographie, Spektroskopie und Massenanalysator/-Spektrometrie,
wobei Chromatographie bevorzugt GC, CE, LC, HPLC und UHPLC umfasst;
wobei Spektroskopie bevorzugt UV/Vis, IR, NIR und NMR umfasst, und
wobei Massenanalysator/-Spektrometrie bevorzugt ESI, Quadrupol-Massenanalysator, Ionenfallen- Massenanalysator, TOF (Flugzeit/Time of Flight)-Massenanalysator, Orbitrap- Massenanalysator, Magnetsektor- Massenanalysator, elektrostatischen Sektor- Massenanalysator, Ionen-Zyklotron-Resonanz (ICR) und Kombinationen davon umfasst,
einschließlich Single Quadrupol (Q) und Triple Quadrupol (QqQ), QqTOF, TOF-TOF, Q-Orbitrap, APCI-QqQ, APCI-QqTOF, MALDI-QqQ, MALDI- QqTOF, and MALDI-TOF-TOF.

## Revendications

1. Utilisation d'une combinaison de métabolites contenus dans un échantillon de sang d'un sujet mammifère, la combinaison de métabolites comprenant
au moins les acides aminés ornithine (Om), thréonine (Thr) et tryptophane (Trp),
et
au moins une acylcarnitine, qui est l'acétylcarnitine (C2 ou AC.2.0),
dans le diagnostic *in vitro* ou *ex vivo* du cancer du sein primaire
comprenant la détection de la présence des métabolites dans ledit échantillon et sa comparaison avec un échantillon de contrôle.

2. Utilisation selon la revendication 1, comprenant en outre les acides aminés choisis parmi le glutamate (Glu), l'aspartate (Asp) et l'histidine (His).

3. Utilisation selon la revendication 1 ou 2, comprenant en outre des acylcarnitines choisies parmi la propionylcarnitine (C3), l'hexanoylcarnitine (AC.6.0), la dodécanoylcarnitine (AC.12.0) et l'hexadécénoylcarnitine. (AC.16.1).

4. Utilisation selon l'une quelconque des revendications 1 à 3, comprenant en outre au moins une amine biogène, de préférence le sulfoxyde de méthionine (Met-SO).

5. Utilisation selon l'une quelconque des revendications 1 à 4, dans laquelle l'échantillon de sang est du sang total, du plasma ou du sérum.

6. Utilisation selon l'une quelconque des revendications 1 à 5, dans laquelle le sujet mammifère est un humain.

7. Utilisation selon l'une quelconque des revendications 1 à 6, dans laquelle les métabolites sont mesurés sur la base d'une méthode analytique quantitative, de préférence la chromatographie, la spectroscopie et les analyseurs de masse/spectrométrie.

8. Utilisation selon l'une quelconque des revendications 1 à 6, dans laquelle les métabolites sont mesurés en utilisant une méthode à base d'anticorps, telle qu'un ELISA.

9. Utilisation selon la revendication 7, dans laquelle la chromatographie comprend les CG, EC, CL, CLHP et CLUHP.

10. Utilisation selon la revendication 7, dans laquelle la spectroscopie comprend les UV/Vis, IR, NIR et RMN.

11. Utilisation selon la revendication 7, dans laquelle les analyseurs de masse/spectrométrie comprennent l'ESI, les analyseurs de masse quadripolaires, les analyseurs de masse à piège ionique, l'analyseur de masse TOF (temps de vol), l'analyseur de masse Orbitrap, l'analyseur de masse à secteur magnétique, l'analyseur de masse à secteur électrostatique, la résonnance cyclotronique ionique (ICR) et des combinaisons de ceux-ci,
notamment le simple quadripôle (Q) et triple quadripôle (QqQ), QqTOF, TOF-TOF, Q-Orbitrap, APCI-QqQ, APCI-QqTOF, MALDI-QqQ, MALDI-QqTOF et MALDI-TOF-TOF.

12. Méthode *in vitro* ou *ex vivo* pour le diagnostic du cancer du sein primaire, comprenant les étapes suivantes :
(a) fournir un échantillon de sang d'un sujet mammifère,
(b) déterminer la quantité d'une combinaison de métabolites, la combinaison de métabolites étant telle que définie dans l'une quelconque des revendications 1 à 4, et
(c) comparer ladite quantité déterminée en (b) avec un échantillon de contrôle.

13. Méthode selon la revendication 12, dans laquelle l'échantillon de sang est du sang total, du plasma ou du sérum ; et/ou dans laquelle le sujet mammifère est un humain.

14. Méthode selon la revendication 12 ou 13, dans laquelle les métabolites sont mesurés sur la base d'une méthode analytique quantitative,
de préférence la chromatographie, la spectroscopie et les analyseurs de masse/spectrométrie,
dans laquelle la chromatographie comprend de préférence les CG, EC, CL, CLHP et CLUHP ;
dans laquelle la spectroscopie comprend de préférence les UV/Vis, IR, NIR et RMN ; et
dans laquelle les analyseurs de masse/spectrométrie comprennent de préférence l'ESI, les analyseurs de masse quadripolaires, les analyseurs de masse à piège ionique, l'analyseur de masse TOF (temps de vol), l'analyseur de masse Orbitrap, l'analyseur de masse à secteur magnétique, l'analyseur de masse à secteur électrostatique, la résonnance cyclotronique ionique (ICR) et des combinaisons de ceux-ci,
notamment le simple quadripôle (Q) et triple quadripôle (QqQ), QqTOF, TOF-TOF, Q-Orbitrap, APCI-QqQ, APCI-QqTOF, MALDI-QqQ, MALDI-QqTOF et MALDI-TOF-TOF.
